(19) Europäisches Patentamt
European Patent Office
Office européen des brevets

(11) EP 1 958 947 A1

# (12) EUROPEAN PATENT APPLICATION

(43) Date of publication:
**20.08.2008 Bulletin 2008/34**

(21) Application number: **08151539.7**

(22) Date of filing: **15.02.2008**

(51) Int Cl.:
***C07D 401/04*** *(2006.01)* ***C07D 403/04*** *(2006.01)*
***A61K 31/4166*** *(2006.01)* ***A61K 31/4427*** *(2006.01)*
***A61K 31/506*** *(2006.01)*

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **15.02.2007 IN DE03202007**

(71) Applicant: **Ranbaxy Laboratories Limited**
**Haryana 122 001 (IN)**

(72) Inventors:
- **Chakraborti, Asit, Kumar**
  **700084 West Bengal (IN)**
- **Sarin, Simi**
  **122001 Gurgaon (IN)**
- **Rudrawar, Santosh, Vijaykumar**
  **431511 Maharashtra (IN)**
- **Kumar, Raj**
  **132103 Haryana (IN)**
- **Chankeshwara, Sunay, Vijaykumar**
  **413002 Maharashtra (IN)**
- **Ray, Abhijit**
  **110070 New Delhi (IN)**
- **Dastidar, Sunanda, Ghosh**
  **110044 New Delhi (IN)**

(74) Representative: **Cronin, Brian Harold John**
**CRONIN Intellectual Property**
**Chemin de Précossy 31**
**1260 Nyon (CH)**

## (54) Inhibitors of phosphodiesterase type 4

(57) The present invention relates to inhibitors of phosphodiesterase (PDE) type 4.

Compounds disclosed herein can be useful for treating, preventing, inhibiting or suppressing asthma, arthritis, bronchitis, chronic obstructive pulmonary disease (COPD), psoriasis, allergic rhinitis, shock, atopic dermatitis, Crohn's disease, adult respiratory distress syndrome (ARDS), AIDS, eosinophilic granuloma, allergic conjunctivitis, osteoarthritis, ulcerative colitis or other inflammatory diseases, especially in humans.

Processes for the preparation of disclosed compounds, pharmaceutical compositions containing the disclosed compounds and their use as phosphodiesterase (PDE) type 4 inhibitors are provided.

EP 1 958 947 A1

## Description

### Field of the Invention

The present invention relates to inhibitors of phosphodiesterase (PDE) type 4.

Compounds disclosed herein can be useful for treating, preventing, inhibiting or suppressing asthma, arthritis, bronchitis, chronic obstructive pulmonary disease (COPD), psoriasis, allergic rhinitis, shock, atopic dermatitis, Crohn's disease, adult respiratory distress syndrome (ARDS), AIDS, eosinophilic granuloma, allergic conjunctivitis, osteoarthritis, ulcerative colitis or other inflammatory diseases, especially in humans.

Processes for the preparation of disclosed compounds, pharmaceutical compositions containing the disclosed compounds and their use as phosphodiesterase (PDE) type 4 inhibitors are provided.

### Background of the Invention

It is known that cyclic adenosine-3',5'-monophosphate (cAMP) exhibits an important role of acting as an intracellular secondary messenger (Sutherland et al., Pharmacol. Rev., (1960) 12, 265). Its intracellular hydrolysis to adenosine 5'-monophosphate (AMP) causes number of inflammatory conditions which are not limited to psoriasis, allergic rhinitis, shock, atopic dermatitis, Crohn's disease, adult respiratory distress syndrome (ARDS), eosinophilic granuloma, allergic conjunctivitis, osteoarthritis or ulcerative colitis. The most important role in the control of cAMP (as well as of cGMP) levels is played by cyclic nucleotide phosphodiesterases (PDE) which represent a biochemically and functionally, highly variable superfamily of enzymes. Eleven distinct families with more than 25 gene products are currently recognized. Although PDE 1, PDE 2, PDE 3, PDE 4, and PDE 7 all use cAMP as a substrate, only the PDE 4 and PDE 7 types are highly selective for hydrolysis of cAMP. Inhibitors of PDE, particularly the PDE 4 inhibitors, such as rolipram or Ro-1724 are therefore known as cAMP-enhancers. Immune cells contain type 4 and type 3 PDE, the PDE 4 type being prevalent in human mononuclear cells. Thus the inhibition of phosphodiesterase type 4 has been a target for modulation and, accordingly, for therapeutic intervention in a range of disease processes.

The initial observation that xanthine derivatives, theophylline and caffeine inhibit the hydrolysis of cAMP led to the discovery of the required hydrolytic activity in the cyclic nucleotide phosphodiesterase (PDE) enzymes. Distinct classes of PDEs have been recognized (Beavo et al., TIPS, (1990) 11,150), and their selective inhibition has led to improved drug therapy (Nicholus, et al. TIPS, (1991) 12, 19). Thus it was recognized that inhibition of PDE 4 could lead to inhibition of inflammatory mediator release (Verghese et. al, J. Mol. Cell. Cardiol, (1989) 12 (Suppl.II), S 61) and airway smooth muscle relaxation.

WO 01/19798 discloses inhibitors of Factor Xa. WO 2005/016862 discloses substituted arylalkanoic acid derivatives, having superior suppressing action on prostaglandin production and leukotriene production. WO 02/76946 discloses pyridine derivatives, described as functional blockers of human vanilloid receptor 1 (hVR1). WO 2004/064835 discloses use of substituted benzimidazolones, benzoxazolones and benzothiazolones, described as ion channel modulating agents. WO 00/34248 discloses benzimidazolone, benzoxazolone or benzothiazolone derivatives, described as ion channel modulating agents. WO 00/66564 discloses benzimidazolones and analogues as progesterone receptor ligands. WO 93/08180 discloses benzoxazole, benzothiazole and benzimidazole derivatives, described as fungicides. WO 2004/096130 discloses heterocyclic compounds, described as inhibitors of Akt kinase activity. WO 02/14275 discloses heterocyclic sulfonamide derivatives, described as useful for potentiating glutamate receptor function.

### Summary of the Invention

The present invention provides inhibitors of phosphodiesterase (PDE) type 4, which can be used for treating, preventing, inhibiting or suppressing asthma, arthritis, bronchitis, chronic obstructive pulmonary disease (COPD), psoriasis, allergic rhinitis, shock, atopic dermatitis, Crohn's disease, adult respiratory distress syndrome (ARDS), AIDS, eosinophilic granuloma, allergic conjunctivitis, osteoarthritis, ulcerative colitis or other inflammatory diseases. Processes for the synthesis of these compounds are also provided.

Pharmaceutically acceptable salts, pharmaceutically acceptable solvates, enantiomers, diastereomers or N-oxides of these compounds having the same type of activity are also provided.

Pharmaceutical compositions containing the compounds, along with pharmaceutically acceptable carriers, excipients or diluents and optionally containing one or more other compounds, are provided, which can be used for treating, preventing, inhibiting or suppressing asthma, arthritis, bronchitis, chronic obstructive pulmonary disease (COPD), psoriasis, allergic rhinitis, shock, atopic dermatitis, Crohn's disease, adult respiratory distress syndrome (ARDS), AIDS, eosinophilic granuloma, allergic conjunctivitis, osteoarthritis, ulcerative colitis or other inflammatory diseases.

The present invention encompasses compounds having the structure of Formula I,

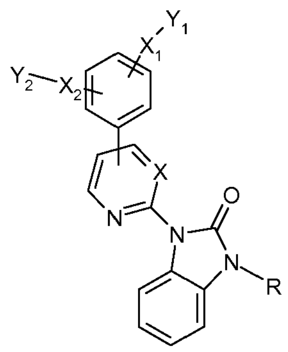

Formula I

and its pharmaceutically acceptable salts, pharmaceutically acceptable solvates, enantiomers, diastereomers or N-oxides, wherein

$Y_1$ and $Y_2$ independently can be hydrogen, alkyl, cycloalkyl, heterocyclyl, heteroaryl, aralkyl, heterocyclylalkyl or heteroarylalkyl;

$X_1$ and $X_2$ independently can be oxygen or sulphur;

X can be nitrogen or carbon;

R can be hydrogen, alkyl, alkenyl, alkynyl, cycloalkyl, aryl, aralkyl, heteroaryl, heterocyclyl, heteroarylalkyl, heterocyclylalkyl, $-COR_x$, $-NR_xR_y$, carboxy, or $-C(=X_3)NR_xR_y$; and

$R_x$ and $R_y$ independently can be hydrogen, alkyl, alkenyl, cycloalkyl, aryl, heterocyclyl, heteroaryl, aralkyl, heterocyclylalkyl or heteroarylalkyl; $R_x$ and $R_y$ may also join together to form cycloalkyl or heterocyclyl ring and $X_3$ can be oxygen or sulfur.

The following definitions apply to terms as used herein.

The term "alkyl," unless otherwise specified, refers to a monoradical branched or unbranched saturated hydrocarbon chain having from 1 to 20 carbon atoms. Alkyl groups can be optionally interrupted by atom(s) or group(s) independently selected from oxygen, sulfur, a phenylene, sulphinyl, sulphonyl group or $-NR_\alpha-$, wherein $R_\alpha$ can be hydrogen, alkyl, cycloalkyl, alkenyl, cycloalkenyl, alkynyl, aryl, acyl, aralkyl, $-C(=O)OR_\lambda$, $SO_mR_\psi$ or $-C(=O)NR_\lambda R_\pi$. This term can be exemplified by groups such as methyl, ethyl, n-propyl, iso-propyl, n-butyl, iso-butyl, sec-butyl, t-butyl, n-pentyl, isopentyl, neopentyl, n-hexyl, n-decyl, tetradecyl, and the like. Alkyl groups may be substituted further with one or more substituents selected from alkenyl, alkynyl, alkoxy, cycloalkyl, cycloalkenyl, acyl, acylamino, acyloxy, alkoxycarbonylamino, azido, cyano, halogen, hydroxy, keto, oxo, thiocarbonyl, carboxy, carboxyalkyl, aryl, heterocyclyl, heteroaryl, (heterocyclyl)alkyl, cycloalkoxy, $-CH=N-O(C_{1-6}alkyl)$, $-CH=N-NH(C_{1-6}alkyl)$, $-CH=N-NH(C_{1-6}alkyl)-C_{1-6}alkyl$, arylthio, thiol, alkylthio, aryloxy, nitro, aminosulfonyl, aminocarbonylamino, $-NHC(=O)R_\lambda$, $-NR_\lambda R_\pi$, $-C(=O)NR_\lambda R_\pi$, $-NHC(=O)NR_\lambda R_\pi$, -C(=O)heteroaryl, C(=O)heterocyclyl, $-O-C(=O)NR_\lambda R_\pi$ {wherein $R_\lambda$ and $R_\pi$ are independently selected from hydrogen, halogen, hydroxy, alkyl, alkenyl, alkynyl, alkenyl, alkoxy, cycloalkyl, cycloalkenyl, aryl, aralkyl, heterocyclyl, heteroaryl, heterocyclylalkyl, heteroarylalkyl or carboxy}, nitro or $-SO_mR_\psi$ (wherein m is an integer from 0-2 and $R_\psi$ is hydrogen, alkyl, alkenyl, alkynyl, cycloalkyl, aralkyl, aryl, heterocyclyl, heteroaryl, heteroarylalkyl or heterocyclylalkyl). Unless otherwise constrained by the definition, alkyl substituents may be further substituted by 1-3 substituents selected from alkyl, alkenyl, alkynyl, carboxy, $-NR_\lambda R_\pi$, $-C(=O)NR_\lambda R_\pi$, $-OC(=O)NR_\lambda R_\pi$, $-NHC(=O)NR_\lambda R_\pi$, hydroxy, alkoxy, halogen, $CF_3$, cyano, and $-SO_mR_\psi$; or an alkyl group also may be interrupted by 1-5 atoms of groups independently selected from oxygen, sulfur or $-NR_\alpha-$ (wherein $R_\alpha$, $R_\lambda$, $R_\pi$, m and $R_\psi$, are the same as defined earlier). Unless otherwise constrained by the definition, all substituents may be substituted further by 1-3 substituents selected from alkyl, alkenyl, alkynyl, carboxy, carboxyalkyl, $-NR_\lambda R_\pi$, $-C(=O)NR_\lambda R_\pi$, $-O-C(=O)NR_\lambda R_\pi$, hydroxy, alkoxy, halogen, $CF_3$, cyano, and $-SO_mR_\psi$ (wherein $R_\lambda$, $R_\pi$, m and $R_\psi$ are the same as defined earlier); or an alkyl group as defined above that has both substituents as defined above and is also interrupted by 1-5 atoms or groups as defined above.

The term "alkylene," as used herein, refers to a diradical branched or unbranched saturated hydrocarbon chain having from 1 to 6 carbon atoms and one or more hydrogen can optionally be substituted with alkyl, hydroxy, halogen or oximes. This term can be exemplified by groups such as methylene, ethylene, propylene isomers (*e.g.*, $-CH_2CH_2CH_2$ and $-CH(CH_3)CH_2$) and the like. Alkylene may further be substituted with one or more substituents such as alkyl, alkenyl, alkynyl, alkoxy, cycloalkyl, acyl, acylamino, acyloxy, alkoxycarbonylamino, azido, cyano, halogen, hydroxy, oxo, thiocarbonyl, carboxy, arylthio, thiol, alkylthio, aryloxy, heteroaryloxy, aminosulfonyl, $-COOR_\psi$, $-NHC(=O)R_\lambda$, $-NR_\lambda R_\pi$, $-C(=O)NR_\lambda R_\pi$, $-NHC(=O)NR_\lambda R_\pi$, -C(=O)heteroaryl, C(=O)heterocyclyl, $-O-C(=O)NR_\lambda R_\pi$, nitro, $-S(O)_mR_\lambda$ (wherein $R_\lambda$, $R_\pi$, m and $R_\psi$, are the same as defined earlier). Unless otherwise constrained by the definition, all substituents may be further substituted by 1-3 substituents chosen from alkyl, alkenyl, alkynyl, carboxy, $-COOR_\psi$, $-NR_\lambda R_\pi$, $-C(=O)NR_\lambda R_\pi$,

-OC(=O)NR$_\lambda$R$_\pi$, -NHC(=O)NR$_\lambda$R$_\pi$, hydroxy, alkoxy, halogen, $CF_3$, cyano, and -S(O)$_m$R$_\psi$ (wherein R$_\lambda$, R$_\pi$, m and R$_\psi$ are the same as defined earlier). Alkylene can also be optionally interrupted by 1-5 atoms of groups independently chosen from oxygen, sulfur and -NR$_\alpha$ (wherein R$_\alpha$ is the same as defined earlier). Unless otherwise constrained by the definition, all substituents may be further substituted by 1-3 substituents selected from hydrogen, alkyl, cycloalkyl, alkenyl, alkynyl, aryl, acyl, aralkyl, alkoxy, hydroxy, carboxy, -C(=O)OR$_\psi$, halogen, $CF_3$, cyano, -NR$_\lambda$R$_\pi$, -S(O)$_m$R$_\psi$, -C(=O)NR$_\lambda$R$_\pi$, -OC (=O)NR$_\lambda$R$_\pi$, -CONH-, -C=O or -C=NOH (wherein R$_\lambda$, R$_\pi$, m and R$_\psi$ are the same as defined earlier).

The term "alkenyl," unless otherwise specified, refers to a monoradical of a branched or unbranched unsaturated hydrocarbon group having from 2 to 20 carbon atoms with cis, trans or geminal geometry. Alkenyl groups can be optionally interrupted by atom(s) or group(s) independently chosen from oxygen, sulfur, phenylene, sulphinyl, sulphonyl and -NR$_\alpha$- (wherein R$_\alpha$ is the same as defined earlier). In the event that alkenyl is attached to a heteroatom, the double bond cannot be alpha to the heteroatom. Alkenyl groups may be substituted further with one or more substituents selected from alkyl, alkenyl, alkynyl, alkoxy, cycloalkyl, cycloalkenyl, acyl, acylamino, acyloxy, -NHC(=O)R$_\lambda$, -NR$_\lambda$R$_\pi$, -C(=O)NR$_\lambda$R$_\pi$, -NHC (=O)NR$_\lambda$R$_\pi$, -O-C(=O)NR$_\lambda$R$_\pi$, alkoxycarbonylamino, azido, cyano, halogen, hydroxy, oxo, keto, carboxyalkyl, thiocarbonyl, carboxy, arylthio, thiol, alkylthio, aryl, aralkyl, aryloxy, heterocyclyl, heteroaryl, heterocyclyl alkyl, heteroaryl alkyl, aminosulfonyl, aminocarbonylamino, alkoxyamino, hydroxyamino, alkoxyamino, nitro or SO$_m$R$_\psi$ (wherein R$_\lambda$, R$_\pi$, m and R$_\psi$ are as defined earlier). Unless otherwise constrained by the definition, alkenyl substituents optionally may be substituted further by 1-3 substituents selected from alkyl, alkenyl, alkynyl, carboxy, hydroxy, alkoxy, halogen, -$CF_3$, cyano, -NR$_\lambda$R$_\pi$, -C(=O)NR$_\lambda$R$_\pi$, -O-C(=O)NR$_\lambda$R$_\pi$ and -SO$_m$R$_\psi$ (wherein R$_\lambda$, R$_\pi$, m and R$_\psi$, are as defined earlier). Groups, such as ethenyl or vinyl ($CH{=}CH_2$), 1-propylene or allyl (-$CH_2CH{=}CH_2$), iso-propylene (-$C(CH_3){=}CH_2$), bicyclo[2.2.1]heptene, and the like, exemplify this term.

The term "alkenylene" unless otherwise specified, refers to a diradical of a branched or unbranched unsaturated hydrocarbon group preferably having from 2 to 6 carbon atoms with cis, trans or geminal geometry. In the event that alkenylene is attached to the heteroatom, the double bond cannot be alpha to the heteroatom. The alkenylene group can be connected by two bonds to the rest of the structure of compound of Formula I. Alkenylene may further be substituted with one or more substituents such as alkyl, alkenyl, alkynyl, alkoxy, cycloalkyl, acyl, acylamino, acyloxy, -NHC(=O)R$_\lambda$, -NR$_\lambda$R$_\pi$, -C(=O)NR$_\lambda$R$_\pi$, -NHC(=O)NR$_\lambda$R$_\pi$,-OC(=O)NR$_\lambda$R$_\pi$ (wherein R$_\lambda$ and R$_\pi$ are the same as defined earlier), alkoxycarbonylamino, azido, cyano, halogen, hydroxy, oxo, thiocarbonyl, carboxy, -COOR$_\psi$ (wherein R$_\psi$ is the same as defined earlier), arylthio, thiol, alkylthio, aryl, aralkyl, aryloxy, heterocyclyl, heteroaryl, heterocyclyl alkyl, heteroaryl alkyl, aminosulfonyl, alkoxyamino, nitro, -S(O)$_m$R$_\psi$ (wherein R$_\psi$ and m are the same as defined earlier). Unless otherwise constrained by the definition, all substituents may optionally be further substituted by 1-3 substituents chosen from alkyl, alkenyl, alkynyl, carboxy, -COOR$_\psi$ (wherein R$_\psi$ is the same as defined earlier), hydroxy, alkoxy, halogen, -$CF_3$, cyano, -NR$_\lambda$R$_\pi$, -C(=O)NR$_\lambda$R$_\pi$, -OC(=O)NR$_\lambda$R$_\pi$ (wherein R$_\lambda$ and R$_\pi$ are the same as defined earlier) and -S(O)$_m$R$_\psi$ (wherein R$_\psi$ and m are the same as defined earlier).

The term "alkynyl," unless otherwise specified, refers to a monoradical of an unsaturated hydrocarbon, having from 2 to 20 carbon atoms. Alkynyl groups can be optionally interrupted by atom(s) or group(s) independently chosen from oxygen, sulfur, phenylene, sulphinyl, sulphonyl and -NR$_\alpha$- (wherein R$_\alpha$ is the same as defined earlier). In the event that alkynyl groups are attached to a heteroatom, the triple bond cannot be alpha to the heteroatom. Alkynyl groups may be substituted further with one or more substituents selected from alkyl, alkenyl, alkoxy, cycloalkyl, cycloalkenyl, acyl, acylamino, acyloxy, alkoxycarbonylamino, azido, cyano, halogen, hydroxy, keto, oxo, thiocarbonyl, carboxy, carboxyalkyl, arylthio, thiol, alkylthio, aryl, aralkyl, aryloxy, aminosulfonyl, aminocarbonylamino, hydroxyamino, alkoxyamino, nitro, heterocyclyl, heteroaryl, heterocyclylalkyl, heteroarylalkyl, -NHC(=O)R$_\lambda$, -NR$_\lambda$R$_\pi$, -NHC(=O)NR$_\lambda$R$_\pi$, -C(=O)NR$_\lambda$R$_\pi$, -O-C(=O)NR$_\lambda$R$_\pi$, or -SO$_m$R$_\psi$ (wherein R$_\lambda$, R$_\pi$, m and R$_\psi$ are the same as defined earlier). Unless otherwise constrained by the definition, alkynyl substituents optionally may be substituted further by 1-3 substituents selected from alkyl, alkenyl, alkynyl, carboxy, carboxyalkyl, hydroxy, alkoxy, halogen, $CF_3$, -NR$_\lambda$R$_\pi$, -C(=O)NR$_\lambda$R$_\pi$, -NHC(=O)NR$_\lambda$R$_\pi$, -C(=O)NR$_\lambda$R$_\pi$, cyano or -SO$_m$R$_\psi$ (wherein R$_\lambda$, R$_\pi$, m and R$_\psi$ are the same as defined earlier).

The term "alkynylene" unless otherwise specified, refers to a diradical of a triply-unsaturated hydrocarbon, preferably having from 2 to 6 carbon atoms. In the event that alkynylene is attached to the heteroatom, the triple bond cannot be alpha to the heteroatom. The alkenylene group can be connected by two bonds to the rest of the structure of compound of Formula I. Alkynylene may further be substituted with one or more substituents such as alkyl, alkenyl, alkoxy, cycloalkyl, acyl, acylamino, acyloxy, alkoxycarbonylamino, azido, cyano, halogen, hydroxy, oxo, thiocarbonyl, carboxy, arylthio, thiol, alkylthio, aryl, aralkyl, aryloxy, aminosulfonyl, nitro, heterocyclyl, heteroaryl, heterocyclyl alkyl, heteroarylalkyl, -NHC(=O)R$_\lambda$, -NR$_\lambda$R$_\pi$, -NHC(=O)NR$_\lambda$R$_\pi$,- C(=O)NR$_\lambda$R$_\pi$, -OC(=O)NR$_\lambda$R$_\pi$ (wherein R$_\lambda$ and R$_\pi$ are the same as defined earlier), -S(O)$_m$R$_\psi$ (wherein R$_\psi$ and m are the same as defined earlier). Unless otherwise constrained by the definition, all substituents may optionally be further substituted by 1-3 substituents chosen from alkyl, alkenyl, alkynyl, carboxy, -COOR$_\psi$ (wherein R$_\psi$ is the same as defined earlier), hydroxy, alkoxy, halogen, $CF_3$, -NR$_\lambda$R$_\pi$, -C(=O) NR$_\lambda$R$_\pi$, -NHC(=O)NR$_\lambda$R$_\pi$, -C(=O)NR$_\lambda$R$_\pi$ (wherein R$_\lambda$ and R$_\pi$ are the same as defined earlier), cyano, and -S(O)$_m$R$_\psi$ (wherein R$_\psi$ and m are the same as defined earlier).

The term "cycloalkyl," unless otherwise specified, refers to cyclic alkyl groups of from 3 to 20 carbon atoms

having a single cyclic ring or multiple condensed rings, which may optionally contain one or more olefinic bonds, unless otherwise constrained by the definition. Such cycloalkyl groups can include, for example, single ring structures, including cyclopropyl, cyclobutyl, cyclooctyl, cyclopentenyl, and the like or multiple ring structures, including adamantanyl, and bicyclo [2.2.1] heptane or cyclic alkyl groups to which is fused an aryl group, for example, indane, and the like. Spiro and fused ring structures can also be included. Cycloalkyl groups may be substituted further with one or more substituents selected from alkyl, alkenyl, alkynyl, alkoxy, cycloalkyl, cycloalkenyl, acyl, acylamino, acyloxy, alkoxycarbonylamino, azido, cyano, halogen, hydroxy, oxo, thiocarbonyl, carboxy, carboxyalkyl, arylthio, thiol, alkylthio, aryl, aralkyl, aryloxy, aminosulfonyl, aminocarbonylamino, $-NR_{\lambda}R_{\pi}$, $-NHC(=O)NR_{\lambda}R_{\pi}$, $-NHC(=O)R_{\lambda}$, $-C(=O)NR_{\lambda}R_{\pi}$, $-O-C(=O)NR_{\lambda}R_{\pi}$, nitro, heterocyclyl, heteroaryl, heterocyclylalkyl, heteroarylalkyl or $SO_mR_{\psi}$ (wherein $R_{\lambda}$, $R_{\pi}$, m and $R_{\psi}$ are the same as defined earlier). Unless otherwise constrained by the definition, cycloalkyl substituents optionally may be substituted further by 1-3 substituents selected from alkyl, alkenyl, alkynyl, carboxy, hydroxy, alkoxy, halogen, $CF_3$, $-NR_{\lambda}R_{\pi}$, $-C(=O)NR_{\lambda}R_{\pi}$, $-NHC(=O)NR_{\lambda}R_{\pi}$, $-OC(=O)NR_{\lambda}R_{\pi}$, cyano or $-SO_mR_{\psi}$ (wherein $R_{\lambda}$, $R_{\pi}$, m and $R_{\psi}$, are the same as defined earlier). "Cycloalkylalkyl" refers to alkyl-cycloalkyl group linked through alkyl portion, wherein the alkyl and cycloalkyl are the same as defined earlier

The term "alkoxy" denotes the group O-alkyl, wherein alkyl is the same as defined above.

The term "aryl," unless otherwise specified, refers to aromatic system having 6 to 14 carbon atoms, wherein the ring system can be mono-, bi- or tricyclic and are carbocyclic aromatic groups. For example, aryl groups include, but are not limited to, phenyl, biphenyl, anthryl or naphthyl ring and the like, optionally substituted with 1 to 3 substituents selected from halogen (e.g., F, Cl, Br, I), hydroxy, alkyl, alkenyl, alkynyl, cycloalkyl, alkoxy, acyl, aryloxy, $CF_3$, cyano, nitro, $COOR_{\psi}$, $NHC(=O)R_{\lambda}$, $-NR_{\lambda}R_{\pi}$, $-C(=O)NR_{\lambda}R_{\pi}$, $-NHC(=O)NR_{\lambda}R_{\pi}$, $-O-C(=O)NR_{\lambda}R_{\pi}$, $-SO_mR_{\psi}$, carboxy, heterocyclyl, heteroaryl, heterocyclylalkyl, heteroarylalkyl or amino carbonyl amino, mercapto, haloalkyl, optionally substituted aryl, optionally substituted heterocyclylalkyl, thioalkyl, $-CONHR_{\pi}$, $-OCOR_{\pi}$, $-COR_{\pi}$, $-NHSO_2R_{\pi}$ or $-SO_2NHR_{\pi}$ (wherein $R_{\lambda}$, $R_{\pi}$, m and $R_{\psi}$, are the same as defined earlier). Aryl groups optionally may be fused with a cycloalkyl group, wherein the cycloalkyl group may optionally contain heteroatoms selected from O, N or S. Groups such as phenyl, naphthyl, anthryl, biphenyl, and the like exemplify this term.

The term "aralkyl," unless otherwise specified, refers to alkyl-aryl linked through an alkyl portion (wherein alkyl is as defined above) and the alkyl portion contains 1-6 carbon atoms and aryl is as defined below. Examples of aralkyl groups include benzyl, ethylphenyl, propylphenyl, naphthylmethyl and the like.

The term "carboxy," unless otherwise specified, refers to $-C(=O)OR_2$ (wherein $R_2$ is hydrogen, alkyl, alkenyl, alkynyl, aryl, cycloalkyl, aralkyl, heteroarylalkyl or heterocyclylalkyl).

The term "heteroaryl," unless otherwise specified, refers to an aromatic ring structure containing 5 or 6 ring atoms or a bicyclic or tricyclic aromatic group having from 8 to 10 ring atoms, with one or more heteroatom(s) independently selected from N, O or S optionally substituted with 1 to 4 substituent(s) selected from halogen (e.g., F, Cl, Br, I), hydroxy, alkyl, alkenyl, alkynyl, cycloalkyl, acyl, carboxy, aryl, alkoxy, aralkyl, cyano, nitro, heterocyclyl, heteroaryl, $-NR_{\lambda}R_{\pi}$, CH=NOH, $-(CH_2)_wC(=O)R_{\eta}$ {wherein w is an integer from 0-4 and $R_{\eta}$ is hydrogen, hydroxy, $OR_{\lambda}$, $NR_{\lambda}R_{\pi}$, $-NHOR_{\omega}$ or -NHOH}, $-C(=O)NR_{\lambda}R_{\pi}$ $-NHC(=O)NR_{\lambda}R_{\pi}$, $-SO_mR_{\psi}$, $-O-C(=O)NR_{\lambda}R_{\pi}$, $-O-C(=O)R_{\lambda}$, or $-O-C(=O)OR_{\lambda}$ (wherein m, $R_{\psi}$, $R_{\lambda}$ and $R_{\pi}$ are as defined earlier and $R_{\omega}$ is alkyl, cycloalkyl, aryl, heteroaryl, heterocyclyl, heteroarylalkyl or heterocyclylalkyl). Unless otherwise constrained by the definition, the substituents are attached to a ring atom, *i.e.,* carbon or heteroatom in the ring. Examples of heteroaryl groups include oxazolyl, imidazolyl, pyrrolyl, 1,2,3-triazolyl, 1,2,4-triazolyl, tetrazolyl, thiazolyl, oxadiazolyl, benzoimidazolyl, thiadiazolyl, pyridinyl, pyridazinyl, pyrimidinyl, pyrazinyl, thienyl, isoxazolyl, triazinyl, furanyl, benzofuranyl, indolyl, benzthiazinyl, benzthiazinonyl, benzoxazinyl, benzoxazinonyl, quinazonyl, carbazolyl phenothiazinyl, phenoxazinyl, benzothiazolyl, benzoxazolyl, 3H-imidazo[4,5-b]pyridinyl, 1H-pyrrolo[2,3-b]pyridinyl and the like.

The term "heterocyclyl," unless otherwise specified, refers to a non-aromatic monocyclic or bicyclic cycloalkyl group having 5 to 10 atoms wherein 1 to 4 carbon atoms in a ring are replaced by heteroatoms selected from O, S or N, and optionally are benzofused or fused heteroaryl having 5-6 ring members and/or optionally are substituted, wherein the substituents are selected from halogen (e.g., F, Cl, Br, I), hydroxy, alkyl, alkenyl, alkynyl, cycloalkyl, acyl, optionally substituted aryl, alkoxy, alkaryl, cyano, nitro, oxo, carboxy, optionally substituted heterocyclyl, optionally substituted heterocyclylalkyl, optionally substituted heteroaryl, $-O-C(=O)R_{\lambda}$, $-O-C(=O)OR_{\lambda}$, $-C(=O)NR_{\lambda}R_{\pi}$, $SO_mR_{\psi}$, $-O-C(=O)NR_{\lambda}R_{\pi}$, $-NHC(=O)NR_{\lambda}R_{\pi}$, $-NR_{\lambda}R_{\pi}$, mercapto, haloalkyl, thioalkyl, $-COOR_{\psi}$, $-COONHR_{\lambda}$, $-COR_{\lambda}$, $-NHSO_2R_{\lambda}$ or $SO_2NHR_{\lambda}$ (wherein m, $R_{\psi}$, $R_{\lambda}$ and $R_{\pi}$ are as defined earlier) or guanidine. Heterocyclyl can optionally include rings having one or more double bonds. Such ring systems can be mono-, bi- or tricyclic. Carbonyl or sulfonyl group can replace carbon atom(s) of heterocyclyl. Unless otherwise constrained by the definition, the substituents are attached to the ring atom, *i.e.,* carbon or heteroatom in the ring. Also, unless otherwise constrained by the definition, the heterocyclyl ring optionally may contain one or more olefinic bond(s). Examples of heterocyclyl groups include oxazolidinyl, tetrahydrofuranyl, dihydrofuranyl, benzoxazinyl, benzthiazinyl, carbaxolyl, phenoxazinyl, phenothiazinyl, dihydropyridinyl, dihydroisoxazolyl, dihydrobenzofuryl, azabicyclohexyl, thiazolidinyl, dihydroindolyl, pyridinyl, isoindole 1,3-dione, piperidinyl, tetrahydropyranyl, piperazinyl, isoquinolinyl, or piperazinyl and the like.

"Heteroarylalkyl," unless otherwise specified, refers to an alkyl-heteroaryl group, wherein the alkyl and heteroaryl portions are the same as defined earlier.

"Heterocyclylalkyl," unless otherwise specified, refers to an alkyl-heterocyclyl group, wherein the alkyl and heterocyclyl portions of the group are the same as defined earlier.

The term "acyl" as defined herein refers to -C(=O)$R_1$ wherein $R_1$ is the same as defined earlier.

The term "haloalkyl" refers to a branched or straight chain alkyl group in which at least one hydrogen atom is replaced with a halogen. Examples of haloalkyl include, but are not limited to, chloromethyl, difluoromethyl, trifluoromethyl, 1-fluoro-2-chloro-ethyl, 5-fluoro-hexyl, 3-difluoro-isopropyl, 3-chloro-isobutyl, chlorodifluoromethyl and the like.

The term "substituted amino" unless otherwise specified, refers to a group $-N(R_k)_2$ wherein each $R_k$ is independently selected from the group hydrogen provided that both $R_k$ groups are not hydrogen (defined as "amino"), alkyl, alkenyl, alkynyl, aralkyl, cycloalkyl, aryl, heteroaryl, heterocyclyl, heterocyclylalkyl, heteroarylalkyl, acyl, $S(O)_mR_\psi$ (wherein m and $R_\psi$ are the same as defined above), $-C(=R_v)NR_\lambda R_y$ (wherein $R_v$ is O or S & $R_\lambda$ and $R_y$ are the same as defined earlier) or $NHC(=R_v)NR_yR_\lambda$ (wherein $R_v$, $R_y$ and $R_\lambda$ are the same as defined earlier). Unless otherwise constrained by the definition, all amino substituents may optionally be further substituted by 1-3 substituents chosen from alkyl, aralkyl, cycloalkyl, aryl, heteroaryl, heterocyclyl, carboxy, $-COOR_\psi$ (wherein $R_\psi$ is the same as defined earlier), hydroxy, alkoxy, halogen, $CF_3$, cyano, $-C(=R_v)NR_\lambda R_y$ (wherein $R_v$ is the same as defined earlier), $-O(C=O)NR_\lambda R_y$, $-OC(=R_v)NR_\lambda R_y$ (wherein $R_\lambda$, $R_y$ and $R_v$ are the same as defined earlier), $-S(O)_mR_\psi$ (wherein $R_\psi$ and m are the same as defined above).

The compounds of the present invention can be used for treating, preventing, inhibiting or suppressing asthma, arthritis, bronchitis, chronic obstructive pulmonary disease (COPD), psoriasis, allergic rhinitis, shock, atopic dermatitis, Crohn's disease, adult respiratory distress syndrome (ARDS), AIDS, eosinophilic granuloma, allergic conjunctivitis, osteoarthritis, ulcerative colitis or other inflammatory diseases. Accordingly, the present invention encompasses a method for treating, preventing, inhibiting or suppressing asthma, arthritis, bronchitis, chronic obstructive pulmonary disease (COPD), psoriasis, allergic rhinitis, shock, atopic dermatitis, Crohn's disease, adult respiratory distress syndrome (ARDS), AIDS, eosinophilic granuloma, allergic conjunctivitis, osteoarthritis, ulcerative colitis or other inflammatory diseases, which comprises administering to a mammal, a therapeutically effective amount of a compound or a pharmaceutical composition of the present invention.

Detailed Description of the Invention

The compounds of the present invention may be prepared by the process described herein. Further, the various synthetic steps described herein may be performed in an alternate sequence in order to give the desired compounds.

## Scheme I

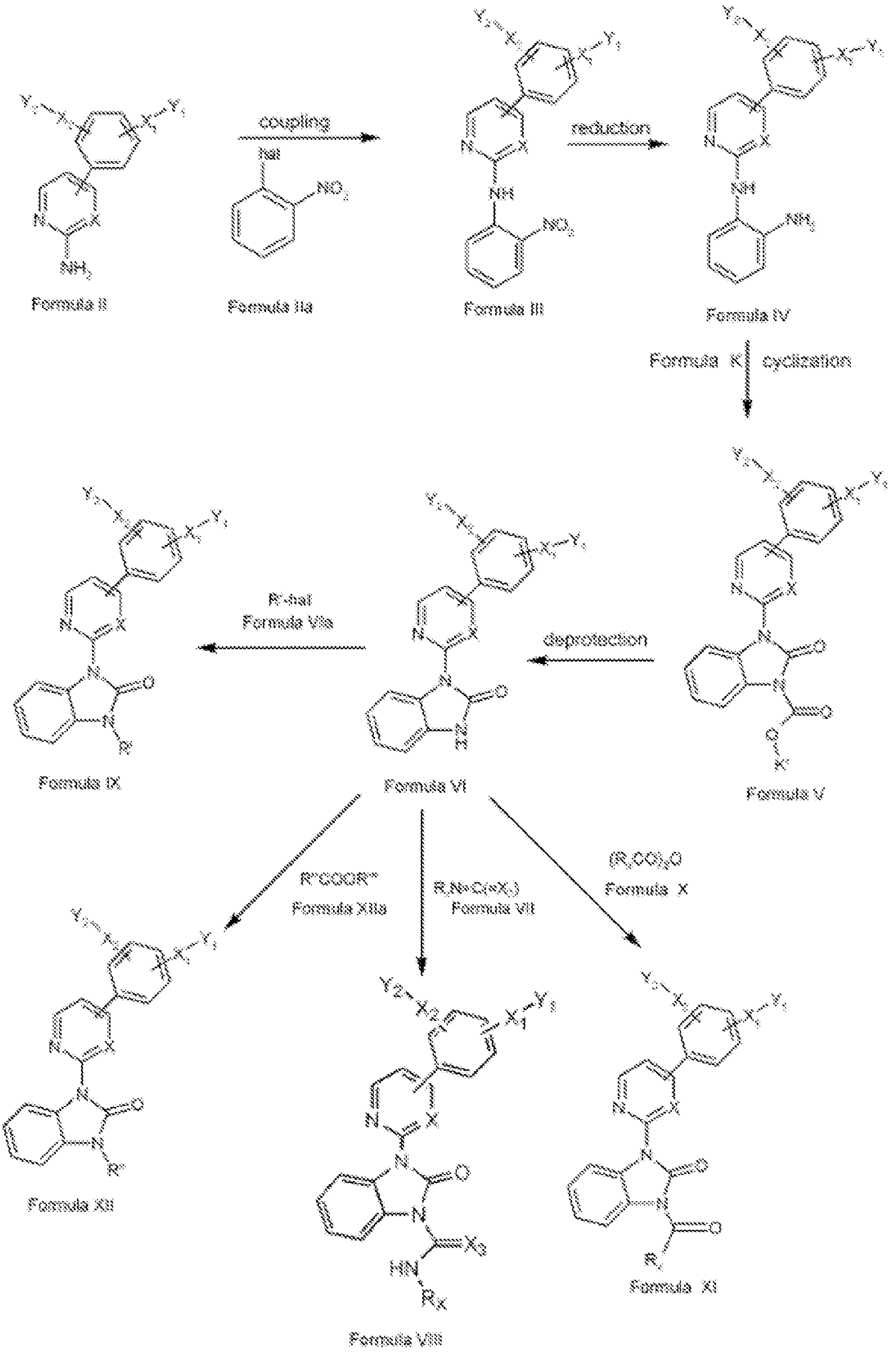
coupling
hal
Formula II
Formula IIa
reduction
Formula III
Formula IV
Formula K
cyclization
Formula V
deprotection
Formula VI
R'-hal
Formula VIIa
Formula IX
R"COOR'"
Formula XIIa
Formula XII
$R_xN{=}C({=}X_3)$
Formula VII
Formula VIII
$(R_xCO)_2O$
Formula X
Formula XI

The compounds of Formulae V, VI, VIII, IX, XI and XII can be prepared by following reaction procedure as depicted in Scheme I, which comprises coupling of a compound of Formula II (wherein $X_1$, $X_2$, $Y_1$, $Y_2$ and X are the same as defined earlier) with a compound of Formula IIa (wherein hal is fluorine, chlorine, bromine or iodine) to give a compound of Formula III, which can undergo reduction to give a compound of Formula IV, which can undergo cyclization by reacting with a compound of Formula K [wherein Formula K is $(CO_2CH_2C_6H_5)_2O$, $(CO_2C(CH_3)_3)_2O$, $(CO_2C(CH_3)_2CHBr_2)_2O$ or $(CO_2C(CH_3)_2CCl_3)_2O$] to give a compound of Formula V [wherein K' is $-CH_2C_6H_5$, - $C(CH_3)_3$, $-C(CH_3)_2CHBr_2$ or $-C(CH_3)_2CCl_3$, respectively], which can be deprotected to give a compound of Formula VI, which can be reacted with

(i) a compound of Formula VIa (wherein hal is the same as defined earlier and R' is alkyl, alkenyl, alkynyl, cycloalkyl, aryl, heteroaryl, heterocyclyl, aralkyl or heteroarylalkyl) to give a compound of Formula IX.
(ii) a compound of Formula VII (wherein $R_x$ is the same as defined earlier and $X_3$ is oxygen or sulfur) to give a compound of Formula VIII.
(iii) a compound of Formula X (wherein $R_x$ is the same as defined earlier) to give a compound of Formula XI.
(iv) a compound of Formula XIIa (wherein R" is haloalkyl and R' is alkyl) to give a compound of Formula XII.

The coupling of a compound of Formula II with a compound of Formula IIa to give a compound of Formula III can be carried out in the presence of a base, for example, sodium hydride, potassium hydride or lithium hydride in a solvent, for example, tetrahydrofuran, dioxane, toluene or iso-propyl alcohol.

The reduction of a compound of Formula III to give a compound of Formula IV can be carried out with reducing agents, for example, palladium on carbon in presence of hydrogen gas or source of hydrogen (for example, ammonium formate solution, formic acid or cyclohexene) in a solvent, for example, methanol, ethanol, iso-propyl alcohol, ethyl acetate, dimethylformamide, tetrahydrofuran, diethylether or dioxane.

The cyclization of a compound of Formula IV with a compound of Formula K to give a compound of Formula V can be carried out in a solvent, for example, acetonitrile, dichloromethane, dichloroethane, chloroform or carbon tetrachloride in the presence of a base, for example, 4-dimethylaminopyridine, *N,N*-diisopropylethylamine, *N*-methylmorpholine or pyridine.

The deprotection of a compound of Formula V [wherein K' is $-C(CH_3)_3$], to give a compound of Formula VI can be carried out in the presence of an alkali metal carbonate, for example, potassium carbonate, sodium carbonate or lithium carbonate or an alkali metal bicarbonate, for example, potassium bicarbonate, lithium bicarbonate or sodium bicarbonate in an alcohol, for example, methanol, ethanol, propanol or isopropylalcohol.

Alternatively, the deprotection of a compound of Formula V [wherein K' is - $C(CH_3)_3$] can be carried out in the presence of trifluro acetic anhydride in dichloromethane.

The deprotection of a compound of Formula V (wherein K' is - $CH_2C_6H_5$) to give a compound of Formula VI can be carried out in the presence of an alkali metal hydroxide, for example, potassium hydroxide, sodium hydroxide or lithium hydroxide in an alcohol, for example, methanol, ethanol, propanol or isopropylalcohol.

Alternatively, the deprotection of a compound of Formula V (wherein K' is - $CH_2C_6H_5$) can be carried out in the presence of a deprotecting agent, for example, palladium on carbon in presence of hydrogen gas or palladium on carbon with a source of hydrogen gas (for example, ammonium formate solution, cyclohexene or formic acid) in a solvent, for example, methanol, ethanol, propanol or isopropylalcohol.

The deprotection of a compound of Formula V (wherein K' is $-C(CH_3)_2CHBr_2$) to give a compound of Formula VI can be carried out in an acidic solution of an alcohol (for example, hydrochloric acid solution of methanol, ethanol, propanol or isopropylalcohol) or trifluoroacetic acid in dichloromethane.

The deprotection of a compound of Formula V (wherein K' is $-C(CH_3)_2CCl_3$) to give a compound of Formula VI can be carried out by a supernucleophile, for example, lithium cobalt (I) phthalocyanine, zinc and acetic acid or cobalt phthalocyanine.

Alternatively, the compound of Formula VI can be prepared by reacting compound of Formula IV with a carbonylating agent (for example, carbonyldiimidazole) in the presence of a base, for example, 4-dimethylaminopyridine, *N,N*-diisopropylethylamine, *N*-methylmorpholine or pyridine.

The reaction of a compound of Formula VI with a compound of Formula VII to give a compound of Formula VIII can be carried out in the presence of a base, for example, 4-dimethylaminopyridine, triethylamine, *N,N*-diisopropylethylamine, *N*-methylmorpholine or pyridine in a solvent, for example, dichloromethane, dichloroethane, carbon tetrachloride or chloroform.

The reaction of a compound of Formula VI with a compound of Formula VIa to give a compound of Formula IX can be carried out in the presence of a base, for example, potassium *tert*-butoxide, sodium hydride, lithium hydride or potassium hydride in a solvent, for example, dimethylformamide, acetonitrile or tetrahydrofuran.

The reaction of a compound of Formula VI with a compound of Formula X to give a compound of Formula XI can be carried out in the presence of a base, for example, potassium *tert*-butoxide, sodium hydride, lithium hydride,

dimethylaminopyridine or potassium hydride in a solvent, for example, *tert*-butanol, dichloromethane, tetrahydrofuran, acetonitrile or dimethylformamide.

The reaction of a compound of Formula VI with a compound of Formula XIIa to give a compound of Formula XII can be carried out in the presence of a base, for example, cesium carbonate, sodium hydride, lithium hydride or potassium hydride in a solvent, for example, dichloromethane, tetrahydrofuran or dimethylformamide.

Particular illustrative compounds which may be prepared by following, for example, the scheme disclosed herein include:

1-{4-[4-(Cyclopentyloxy)-3-methoxyphenyl]pyrimidin-2-yl}-1,3-dihydro-2*H*-benzimidazol-2-one (Compound No. 1),

*tert*-Butyl 3- {4-[4-(cyclopentyloxy)-3-methoxyphenyl]pyrimidin-2-yl}-2-oxo-2,3-dihydro-1*H*-benzimidazole-1-carboxylate (Compound No. 2),

*tert*-Butyl 3-{5-[3-(cyclopentyloxy)-4-methoxyphenyl]pyrimidin-2-yl}-2-oxo-2,3-dihydro-1*H*-benzimidazole-1-carboxylate (Compound No. 3),

*tert*-Butyl 3-[5-(3,4-dimethoxyphenyl)pyrimidin-2-yl]-2-oxo-2,3-dihydro-1*H*-benzimidazole-1-carboxylate (Compound No. 4),

*tert*-Butyl 3-[5-(2,5-dimethoxyphenyl)pyrimidin-2-yl]-2-oxo-2,3-dihydro-1*H*-benzimidazole-1-carboxylate (Compound No. 5),

*tert*-Butyl 3-[4-(3,4-dimethoxyphenyl)pyrimidin-2-yl]-2-oxo-2,3-dihydro-1*H*-benzimidazole-1-carboxylate (Compound No. 6),

1-[5-(3, 4-Dimethoxyphenyl)pyrimidin-2-yl]-1,3-dihydro-2*H*-benzimidazol-2-one (Compound No. 7),

1-[5-(2, 5-Dimethoxyphenyl)pyrimidin-2-yl]-1,3-dihydro-2*H*-benzimidazol-2-one (Compound No. 8),

1-{5-[3-(Cyclopentyloxy)-4-methoxyphenyl]pyrimidin-2-yl}-1,3-dihydro-2*H*-benzimidazol-2-one (Compound No. 9),

1-[4-(3, 4-Dimethoxyphenyl)pyrimidin-2-yl]-1,3-dihydro-2*H*-benzimidazol-2-one (Compound No. 10),

1-(Difluoromethyl)-3-[4-(3,4-dimethoxyphenyl)pyrimidin-2-yl]-1,3-dihydro-2*H*-benzimidazol-2-one (Compound No. 11),

1-[4-(3, 4-Dimethoxyphenyl)pyrimidin-2-yl]-3-ethyl-1,3-dihydro-2*H*-benzimidazol-2-one (Compound No. 12),

1-Cyclopentyl-3-[4-(3,4-dimethoxyphenyl)pyrimidin-2-yl]-1,3-dihydro-2*H*-benzimidazol-2-one (Compound No. 13),

1-Benzyl-3-[4-(3,4-dimethoxyphenyl)pyrimidin-2-yl]-1,3-dihydro-2*H*-benzimidazol-2-one (Compound No. 14),

*tert*-Butyl-3-{5-[3-(cyclopentyloxy)-4-methoxyphenyl]pyridin-2-yl}-2-oxo-2,3-dihydro-1*H*-benzimidazole-1-carboxylate (Compound No. 15),

1-{5-[3-(Cyclopentyloxy)-4-methoxyphenyl]pyridin-2-yl}-3-methyl-1,3-dihydro-2*H*-benzimidazol-2-one (Compound No. 16),

1-Allyl-3- {5-[3-(cyclopentyloxy)-4-methoxyphenyl]pyridin-2-yl}-1,3-dihydro-2*H*-benzimidazol-2-one (Compound No. 17),

1-Benzyl-3- {5-[3-(cyclopentyloxy)-4-methoxyphenyl]pyridin-2-yl}-1,3-dihydro-2*H*-benzimidazol-2-one (Compound No. 18),

1-{5-[3-(Cyclopentyloxy)-4-methoxyphenyl]pyridin-2-yl}-3-ethyl-1,3-dihydro-2*H*-benzimidazol-2-one (Compound No. 19),

1-{5-[3-(Cyclopentyloxy)-4-methoxyphenyl]pyridin-2-yl}-1,3-dihydro-2*H*-benzimidazol-2-one (Compound No. 20),

1-{5-[3-(Cyclopentyloxy)-4-methoxyphenyl]pyridin-2-yl}-3-propyl-1,3-dihydro-2*H*-benzimidazol-2-one (Compound No. 21),

1-Cyclopentyl-3- {5-[3-(cyclopentyloxy)-4-methoxyphenyl]pyridin-2-yl}-1,3-dihydro-2*H*-benzimidazol-2-one (Compound No. 22),

1-{5-[3-(Cyclopentyloxy)-4-methoxyphenyl]pyridin-2-yl}-3-isopropyl-1,3-dihydro-2*H*-benzimidazol-2-one (Compound No. 23),

*tert*-Butyl-3-{4-[3-(cyclopentyloxy)-4-methoxyphenyl]pyrimidin-2-yl}-2-oxo-2,3-dihydro-1*H*-benzimidazole-1-carboxylate (Compound No. 24),

1-{4-[3-(Cyclopentyloxy)-4-methoxyphenyl]pyrimidin-2-yl}-1,3-dihydro-2*H*-benzimidazol-2-one (Compound No. 25),

3-{5-[3-(Cyclopentyloxy)-4-methoxyphenyl]pyrimidin-2-yl}-*N*-isopropyl-2-oxo-2,3-dihydro-1*H*-benzimidazole-1-carboxamide (Compound No. 26),

3-{4-[3-(Cyclopentyloxy)-4-methoxyphenyl]pyrimidin-2-yl}-*N*-isopropyl-2-oxo-2,3-dihydro-1*H*-benzimidazole-1-carboxamide (Compound No. 27),

*N*-Cyclopentyl-3-{5-[3-(cyclopentyloxy)-4-methoxyphenyl]pyrimidin-2-yl}-2-oxo-2,3-dihydro-1*H*-benzimidazole-1-carboxamide (Compound No. 28),

1-Cyclopentyl-3-{5-[3-(cyclopentyloxy)-4-methoxyphenyl]pyrimidin-2-yl}-1,3-dihydro-2*H*-benzimidazol-2-one (Compound No. 29),

1-{5-[3-(Cyclopentyloxy)-4-methoxyphenyl]pyridin-2-yl}-3-(4-methoxybenzyl)-1,3-dihydro-2*H*-benzimidazol-2-one (Compound No. 30),

1-{5-[3-(Cyclopentyloxy)-4-methoxyphenyl]pyridin-2-yl}-3-[4-(trifluoromethyl)benzyl]-1,3-dihydro-2*H*-benzimidazol-2-one (Compound No. 31),

1-{5-[3-(Cyclopentyloxy)-4-methoxyphenyl]pyridin-2-yl}-3-(pyridin-3-ylmethyl)-1,3-dihydro-2*H*-benzimidazol-2-one (Compound No. 32),

1-Acetyl-3-{5-[3-(cyclopentyloxy)-4-methoxyphenyl]pyrimidin-2-yl}-1,3-dihydro-2*H*-benzimidazol-2-one (Compound No. 33),

1-{5-[3-(Cyclopentyloxy)-4-methoxyphenyl]pyrimidin-2-yl}-3-propanoyl-1,3-dihydro-2*H*-benzimidazol-2-one (Compound No. 34),

1-Butanoyl-3-{5-[3-(cyclopentyloxy)-4-methoxyphenyl]pyrimidin-2-yl}-1,3-dihydro-2*H*-benzimidazol-2-one (Compound No. 35),

1-{5-[3-(Cyclopentyloxy)-4-methoxyphenyl]pyrimidin-2-yl}-3-(2-methylpropanoyl)-1,3-dihydro-2*H*-benzimidazol-2-one (Compound No. 36),

1-{5-[3-(Cyclopentyloxy)-4-methoxyphenyl]pyrimidin-2-yl}-3-(phenylcarbonyl)-1,3-dihydro-2*H*-benzimidazol-2-one (Compound No. 37),

1-{5-[3-(Cyclopentyloxy)-4-methoxyphenyl]pyrimidin-2-yl}-3-(2,2-dimethylpropanoyl)-1,3-dihydro-2*H*-benzimidazol-2-one (Compound No. 38),

1-{5-[3-(Cyclopentyloxy)-4-methoxyphenyl]pyridin-2-yl}-3-(phenylcarbonyl)-1,3-dihydro-2*H*-benzimidazol-2-one (Compound No. 39),

1-{5-[3-(Cyclopentyloxy)-4-methoxyphenyl]pyridin-2-yl}-3-propanoyl-1,3-dihydro-2*H*-benzimidazol-2-one (Compound No. 40),

1-Butanoyl-3- {5-[3-(cyclopentyloxy)-4-methoxyphenyl]pyridin-2-yl}-1,3-dihydro-2*H*-benzimidazol-2-one (Compound No. 41),

1-Acetyl-3- {5-[3-(cyclopentyloxy)-4-methoxyphenyl]pyridin-2-yl}-1,3-dihydro-2*H*-benzimidazol-2-one (Compound No. 42),

1-Cyclopentyl-3-{4-[3-(cyclopentyloxy)-4-methoxyphenyl]pyrimidin-2-yl}-1,3-dihydro-2*H*-benzimidazol-2-one (Compound No. 43),

1-{5-[3-(Cyclopentyloxy)-4-methoxyphenyl]pyridin-2-yl}-3-[4-(methylsulfanyl)benzyl]-1,3-dihydro-2*H*-benzimidazol-2-one (Compound No. 44),

*N*-(2-chloroethyl)-3-{5-[3-(cyclopentyloxy)-4-methoxyphenyl]pyridin-2-yl}-2-oxo-2,3-dihydro-1*H*-benzimidazole-1-carboxamide (Compound No. 45),

*N*-(3-chloropropyl)-3-{5-[3-(cyclopentyloxy)-4-methoxyphenyl]pyridin-2-yl}-2-oxo-2,3-dihydro-1*H*-benzimidazole-1-carboxamide (Compound No. 46),

3-{5-[3-(Cyclopentyloxy)-4-methoxyphenyl]pyridin-2-yl}-2-oxo-*N*-prop-2-en-1-yl-2,3-dihydro-1*H*-benzimidazole-1-carboxamide (Compound No. 47),

*N*-benzyl-3-{5-[3-(cyclopentyloxy)-4-methoxyphenyl]pyridin-2-yl}-2-oxo-2,3-dihydro-1*H*-benzimidazole-1-carboxamide (Compound No. 48),

3-{5-[3-(Cyclopentyloxy)-4-methoxyphenyl]pyridin-2-yl}-2-oxo-*N*-pentyl-2,3-dihydro-1*H*-benzimidazole-1-carboxamide (Compound No. 49),

N-cyclopentyl-3- {5-[3-(cyclopentyloxy)-4-methoxyphenyl]pyridin-2-yl}-2-oxo-2,3-dihydro-1*H*-benzimidazole-1-carboxamide (Compound No. 50),

3-{5-[3-(Cyclopentyloxy)-4-methoxyphenyl]pyridin-2-yl}-*N*-heptyl-2-oxo-2,3-dihydro-1*H*-benzimidazole-1-carboxamide (Compound No. 51),

3-{5-[3-(Cyclopentyloxy)-4-methoxyphenyl]pyridin-2-yl}-*N*-(4-methylphenyl)-2-oxo-2,3-dihydro-1*H*-benzimidazole-1-carboxamide (Compound No. 52),

*N*-(4-bromophenyl)-3-{5-[3-(cyclopentyloxy)-4-methoxyphenyl]pyridin-2-yl}-2-oxo-2,3-dihydro-1*H*-benzimidazole-1-carboxamide (Compound No. 53), and

pharmaceutically acceptable salts, pharmaceutically acceptable solvates, enantiomers, diastereomers or N-oxides thereof.

The term "pharmaceutically acceptable" means approved by regulatory agency of the federal or a state government or listed in the U.S. Pharmacopoeia or other generally recognized pharmacopoeia for use in mammals, and more particularly in humans.

The term "pharmaceutically acceptable salts" refers to derivatives of compounds that can be modified by forming their corresponding acid or base salts. Examples of pharmaceutically acceptable salts include, but are not limited to, mineral or organic acids salts of basic residues (such as amines), or alkali or organic salts of acidic residues (such as carboxylic acids), and the like.

The term "pharmaceutically acceptable solvates" refers to solvates with water (i.e. hydrates, hemihydrate or sesquihydrate) or pharmaceutically acceptable solvents, for example, solvates with common organic solvents as ethanol and the like. Such solvates are also encompassed within the scope of the disclosure.

All stereoisomers of the compounds of the invention are contemplated, either in admixture or in pure or substantially pure form. The compounds of the present invention can have asymmetric centers at particular carbon atoms, or their substituents. Consequently, compounds of present invention can exist in enantiomeric or diastereomeric forms or in mixture(s) thereof. The processes for the preparation can utilize racemates, enantiomers, or diastereomers as starting materials. When diastereomeric or enantiomeric products are prepared, they can be separated by conventional methods, for example, chromatographic or fractional crystallization.

When a bond to a substituent is shown to cross a bond connecting two atoms in a ring, then such substituent

may be bonded to any atom on the ring.

In another aspect, the present invention includes pharmaceutical compositions comprising, as an active ingredient, at least one of the disclosed compound or a pharmaceutically acceptable salt, a pharmaceutically acceptable solvate, an enantiomer, a diastereomer or N-oxide, together with a pharmaceutically acceptable carrier, excipient or diluent. Compounds disclosed herein may be administered to a mammal for treatment, prevention, inhibition or suppression of asthma, arthritis, bronchitis, chronic obstructive pulmonary disease (COPD), psoriasis, allergic rhinitis, shock, atopic dermatitis, Crohn's disease, adult respiratory distress syndrome (ARDS), AIDS, eosinophilic granuloma, allergic conjunctivitis, osteoarthritis, ulcerative colitis or other inflammatory diseases, by any route, which effectively transports the active compound to the appropriate or desired site of action such as oral, nasal, pulmonary, transdermal or parenteral (rectal, subcutaneous, intravenous, intraurethral, intramuscular, intranasal). The choice of pharmaceutical carrier, excipient or diluent can be made with regard to the intended route of administration and standard pharmaceutical practice.

Where desired, the compounds of Formula I or their pharmaceutically acceptable salts, pharmaceutically acceptable solvates, enantiomers, diastereomers or N-oxides may be advantageously used in combination with one or more other compounds. Examples of other compounds, which may be used in combination with compounds of Formula I of this invention or their pharmaceutically acceptable salts, pharmaceutically acceptable solvates, enantiomers, diastereomers or N-oxides include corticosteroids, β2- agonists, leukotriene antagonists, 5-lipoxygenase inhibitors, chemokine inhibitors, muscarinic receptor antagonists, p38 MAP kinase inhibitors, anticholinergics, antiallergics, PAF (platelet activating factor) antagonists, EGFR (epidermal growth factor receptor) kinase inhibitors, additional PDE 4 inhibitors, or combinations thereof.

The one or more β2- agonists as described herein may be chosen, for example, from those described in the art. The β2-agonists may include, for example, one or more compounds described in U.S. Patent Nos. 3,705,233; 3,644,353; 3,642,896; 3,700,681; 4,579,985; 3,994,974; 3,937,838; 4,419,364; 5,126,375; 5,243,076; 4,992,474; or 4,011,258.

β2-agonists include, for example, one or more of albuterol, salbutamol, biltolterol, pirbuterol, levosalbutamol, tulobuterol, terbutaline, bambuterol, metaproterenol, fenoterol, salmeterol, carmoterol, arformoterol, formoterol, and their pharmaceutically acceptable salts or solvates thereof.

Corticosteroids as described herein may be chosen, for example, from those described in the art. Corticosteroids may include, for example, one or more compounds described in U.S. Patent Nos 3,312,590; 3,983,233; 3,929,768; 3,721,687; 3,436,389; 3,506,694; 3,639,434; 3,992,534; 3,928,326; 3,980,778; 3,780,177; 3,652,554; 3,947,478; 4,076,708; 4,124,707; 4,158,055; 4,298,604; 4,335,121; 4,081,541; 4,226,862; 4,290,962; 4,587,236; 4,472,392; 4,472,393; 4,242,334; 4,014,909; 4,098,803; 4,619,921; 5,482,934; 5,837,699; 5,889,015; 5,278,156; 5,015,746; 5,976,573; 6,337,324; 6,057,307; 6,723,713; 6,127,353; or 6,180,781.

Corticosteroids may include, for example, one or more of alclometasone, amcinonide, amelometasone, beclometasone, betamethasone, budesonide, ciclesonide, clobetasol, cloticasone, cyclomethasone, deflazacort, deprodone, dexbudesonide, diflorasone, difluprednate, fluticasone, flunisolide, halometasone, halopredone, hydrocortisone, hydrocortisone, methylprednisolone, mometasone, prednicarbate, prednisolone, rimexolone, tixocortol, triamcinolone, tolterodine, oxybutynin, ulobetasol, rofleponide, GW 215864, KSR 592, ST-126, dexamethasone and pharmaceutically acceptable salts, solvates thereof. Preferred corticosteroids include, for example, flunisolide, beclomethasone, triamcinolone, budesonide, fluticasone, mometasone, ciclesonide, and dexamethasone, while budesonide, fluticasone, mometasone, ciclesonide. Examples of possible salts or derivatives include: sodium salts, sulfobenzoates, phosphates, isonicotinates, acetates, propionates, dihydrogen phosphates, palmitates, pivalates, or furoates. In some cases, the corticosteroids may also occur in the form of their hydrates.

Muscarinic receptor antagonists include substances that directly or indirectly block activation of muscarinic cholinergic receptors. Examples include, but are not limited to, quaternary amines (*e.g.*, methantheline, ipratropium, propantheline), tertiary amines (*e.g.*, dicyclomine, scopolamine) and tricyclic amines (e.g., telenzepine). Other muscarinic receptor antagonists include benztropine, hexahydro-sila-difenidol hydrochloride (HHSID hydrochloride), (+/-)-3-quinuclidinyl xanthene-9-carboxylate hemioxalate (QNX-hemioxalate), telenzepine dihydrochloride and atropine.

Anticholinergics include, for example, tiotropium salts, ipratropium salts, oxitropium salts, salts of the compounds known from WO 02/32899: tropenol N-methyl-2,2-diphenylpropionate, scopine N-methyl-2,2-diphenylpropionate, scopine N-methyl-2-fluoro-2,2-diphenylacetate and tropenol N-methyl-2-fluoro-2,2-diphenylacetate; as well as salts of the compounds known from WO 02/32898: tropenol N-methyl-3,3',4,4'-tetrafluorobenzilate, scopine N-methyl-3,3',4,4'-tetrafluorobenzilate, scopine N-methyl-4,4'-dichlorobenzilate, scopine N-methyl-4,4'-difluorobenzilate, tropenol N-methyl-3,3'-difluorobenzilate, scopine N-methyl-3,3'-difluorobenzilate, and tropenol N-ethyl-4,4'-difluorobenzilate, optionally in the form of their hydrates and solvates. By salts are meant those compounds which contain, in addition to the above mentioned cations, as counterion, an anion with a single negative charge selected from among the chloride, bromide, and methanesulfonate.

Antiallergic agents include, for example, epinastine, cetirizine, azelastine, fexofenadine, levocabastine, loratadine, mizolastine, ketotifene, emedastine, dimetindene, clemastine, bamipine, hexachloropheniramine, pheniramine,

doxylamine, chlorophenoxamine, dimenhydrinate, diphenhydramine, promethazine, ebastine, desloratadine, and meclizine. Preferred antiallergic agents include, for example, epinastine, cetirizine, azelastine, fexofenadine, levocabastine, loratadine, ebastine, desloratadine, and mizolastine, epinastine. Any reference to the above-mentioned antiallergic agents also includes any pharmacologically acceptable salts thereof, which may exist.

PAF antagonists include, for example, 4-(2-chlorophenyl)-9-methyl-2-[3-(4-morpholinyl)-3-propanon-1-yl]-6H-thieno[3,2-f][1,2,4]triazolo[4,3-$\alpha$][1,4]diazepine and 6-(2-chlorophenyl)-8,9-dihydro-1-methyl-8-[(4-morpholinyl)carbonyl]-4H,7H-cyclopenta[4.5]thieno[3,2-f][1,2,4]triazolo[4,3-a][1,4]diazepine.

EGFR kinase inhibitors include, for example, 4-[(3-chloro-4-fluorophenyl)amino]-7-(2- {4-[(S)-(2-oxotetrahydrofnran-5-yl)carbonyl]piperazin-1-yl}-ethoxy)-6-[(vinylcarbonyl)amino]quinazoline, 4-[(3-chloro4-fluorophenyl)amino]-7-[4-((S)-6-methyl-2-oxomorpholin-4-yl)butyloxy] -6- [(vinylcarbonyl)amino]quinazo line, 4- [(3 - chloro4-fluorophenyl) amino]-7-[4-((R)-6-methyl-2-oxomorpholin-4-yl)butyloxy]-6-[(vinylcarbonyl)amino]quinazoline, 4-[(3-chloro-4-fluorophenyl)amino]-7-[2-((S)-6-methyl-2-oxomorpholin-4-yl)ethoxy]-6-[(vinylcarbonyl)amino]quinazoline, 4-[(3-chloro-4-fluorophenyl)amino]-6-[(4-{N-[2-(ethoxycarbonyl)ethyl]-N-[(ethoxycarbonyl)methyl]-amino}-1-oxo-2-buten-1-yl)amino]-7-cyclopropylmethoxyquinazoline, 4-[(R)-(1-phenylethyl)amino]-6- {[4-(morpholin-4-yl)-1-oxo-2-buten-1-yl]amino}-7-cyclopropylmethoxyquinazo line, and 4-[(3-chloro-4-fluorophenyl)amino]-6-[3-(morpholin-4-yl)propyloxy]-7-methoxyquinazoline. Any reference to the above-mentioned EGFR kinase inhibitors also includes any pharmacologically acceptable salts thereof which may exist.

p38 MAP kinase inhibitors include, for example, 1-[5-tert-butyl-2-p-tolyl-2H-pyrazol-3-yl]-3-[4-(2-morpholin-4-ylethoxy)naphthalen-1-yl]urea; 1-[5-tert-butyl-2-p-tolyl-2H-pyrazol-3-yl]-3-[4-(2-(1-oxothiomorpholin-4-yl)ethoxy)naphthalen-1-yl]urea; 1-[5-tert-butyl-2-(2-methylpyridin-5-yl)-2H-pyrazol-3-yl]-3-[4-(2-pyridin-4-ylethoxy)naphthalen-1-yl] urea; 1-[5-tert-butyl-2-(2-methoxypyridin-5-yl)-2H-pyrazol-3-yl]-3-[4-(2-morpholin-4-ylethoxy)naphthalen-1-yl]urea; and 1-[5-tert-butyl-2-methyl-2H-pyrazol-3-yl]-3-[4-(2-morpholin-4-ylethoxy)naphthalen-1-yl]urea disclosed in WO06021848; 4-[7-Oxo-8-(tetrahydro-pyran-4-yl)-6-o-tolyl-7,8-dihydro-pyrido[2,3-d]pyrimidin-2-ylamino]-piperidine-1-carboxylic acid tert-butyl ester; Hydrochloride salt of 2-(Piperidin-4-ylamino)-8-(tetrahydro-pyran-4-yl)-6-o-tolyl-8H-pyrido[2,3-d]pyrimidin-7-one; 2-(1-Methanesulfonyl-piperidin-4-ylamino)-8-(tetrahydro-pyran-4-yl)-6-o-tolyl-8H-pyrido[2,3-d]pyrimidin-7-one; 2-(1-Benzyl-piperidin-4-ylamino)-8-(tetrahydro-pyran-4-yl)-6-o-tolyl-8H-pyrido[2,3-d]pyrimidin-7-one; 2-(1-Methyl-piperidin-4-ylamino)-8-(tetrahydro-pyran-4-yl)-6-o-tolyl-8H-pyrido[2,3-d]pyrimidin-7-one; 2-(4-Methyl-piperazin-1-ylamino)-8-(tetrahydro-pyran-4-yl)-6-o-tolyl-8H-pyrido[2,3-d]pyrimidin-7-one; 4-[6-(2-Chloro-phenyl)-7-oxo-8-(tetrahydro-pyran-4-yl)-7,8-dihydro-pyrido[2,3-d]pyrimidin-2-ylamino]-piperidine-1-carboxylic acid tert-butyl ester; 2-(Piperidin-1-ylamino)-8-(tetrahydro-pyran-4-yl)-6-o-tolyl-8H-pyrido[2,3-d]pyrimidin-7-one; 2-Cyclobutylamino-8-(tetrahydro-pyran-4-yl)-6-o-tolyl-8H-pyrido[2,3-d]pyrimidin-7-one; 2-(1-Acetyl-piperidin-4-ylamino)-8-(tetrahydro-pyran-4-yl)-6-o-tolyl-8H-pyrido[2,3-d]pyrimidin-7-one; 2-(1-Benzoyl-piperidin-4-ylamino)-8-(tetrahydro-pyran-4-yl)-6-o-tolyl-8H-pyrido[2,3-d]pyrimidin-7-one; 2-(1-Benzoyl-piperidin-4-ylamino)-8-(tetrahydro-pyran-4-yl)-6-o-tolyl-8H-pyrido [2,3-d]pyrimidin-7-one; 4-[7-Oxo-8-(tetrahydro-pyran-4-yl)-6-o-tolyl-7,8-dihydro-pyrido[2,3-d]pyrimidin-2-ylamino]-piperidine-1-carboxylic acid (4-fluoro-phenyl)-amide; 2-(1-Ethanesulfonyl-piperidin-4-ylamino)-8-(tetrahydro-pyran-4-yl)-6-o-tolyl-8H-pyrido [2,3-d] pyrimidin-7-one; 4-[7-Oxo-8-(tetrahydro-pyran-4-yl)-6-o-tolyl-7,8-dihydro-pyrido[2,3-d]pyrimidin-2-ylamino]-piperidine-1-carbothioic acid (4-fluoro-phenyl)-amide; 4-[7-Oxo-8-(tetrahydropyran-4-yl)-6-o-tolyl-7,8-dihydro-pyrido[2,3-d]pyrimidin-2-ylamino]-piperidine-1-carboxylic acid (4-trifluoromethyl-phenyl)-amide; 2-[4-(Propane-2-sulfonyl)-piperazin-1-ylamino]-8-(tetrahydro-pyran-4-yl)-6-o-tolyl-8H-pyrido[2,3-d]pyrimidin-7-one; 4-[7-Oxo-8-(tetrahydro-pyran-4-yl)-6-o-tolyl-7,8-dihydro-pyrido[2,3-d]pyrimidin-2-ylamino]-piperazine-1-carboxylic acid propylamide; 4-[7-Oxo-8-(tetrahydro-pyran-4-yl)-6-o-tolyl-7,8-dihydro-pyrido[2,3-d]pyrimidin-2-ylamino]-piperazine-1-carboxylic acid ((R)-1,2-dimethyl-propyl)-amide; 4-[7-Oxo-8-(tetrahydro-pyran-4-yl)-6-o-tolyl-7,8-dihydro-pyrido[2,3-d]pyrimidin-2-ylamino]-piperazine-1-carboxylic acid cyclohexylamide; 4-[7-Oxo-8-(tetrahydro-pyran-4-yl)-6-o-tolyl-7,8-dihydro-pyrido[2,3-d]pyrimidin-2-ylamino]-piperazine-1-carboxylic acid (4-fluoro-phenyl)-amide; 4-[7-Oxo-8-(tetrahydro-pyran-4-yl)-6-o-tolyl-7,8-dihydro-pyrido[2,3-d]pyrimidin-2-ylamino]-piperazine-1-carboxylic acid cyclopentyl methyl-amide; and the compounds; which are disclosed in WO 2006/016237, WO 2006/056863, WO 2006/117657 and WO 2006/082492. Any reference to the above mentioned p38 kinase inhibitors also includes any pharmacologically acceptable salts thereof which may exist.

Additional PDE 4 inhibitors include, for example, enprofylline, roflumilast, oglemilast, ariflo, Bay-198004, CP-325,366, BY343, D-4396 (Sch-351591), V-11294A, Z-15370, and AWD-12-281. Preferred PDE 4 inhibitors are selected from enprofylline, roflumilast, ariflo, Z15370, and AWD-12-281. Any reference to the above mentioned PDE 4 inhibitors also includes any pharmacologically acceptable salts thereof which may exist.

The leukotriene antagonist can be selected, for example, from compounds not limited to those described in U.S. Patent Nos. 5,565,473; 5,583,152; 4,859,692; or 4,780,469.

Examples of leukotriene antagonist include, but are not limited to, montelukast, zafirlukast, pranlukast and pharmaceutically acceptable salts thereof.

5-Lipoxygenase inhibitors can be selected, for example, from the compounds disclosed in U.S. Patent Nos. 4,826,868 and 4,873,259, or European Patent Nos. 0 419 049, 0 542 356 or 0 542 355. Examples may include but are not limited to atreleuton, zyflo (zileuton), ABT-761, fenleuton or tepoxalin.

Chemokine inhibitors can be selected from, for example, the compounds disclosed in European Patent Nos. 0 287 436, 0 389 359, 0 988 292, or WO 02/26723 or WO 01/90106.

Examples of chemokine inhibitors include, but are not limited to AMD3100, AZD 8309, BX-471, GW-766994, UK-427857, CP-481715, UK-107543, UK-382055 or UK-395859.

Examples set forth below demonstrate the synthetic procedures for the preparation of the representative compounds. The examples are provided to illustrate particular aspect of the disclosure and do not constrain the scope of the present invention as defined by the claims.

## Experimental

Example 1: Synthesis of *tert*-butyl 3-14-[3-(cyclopentyloxy)-4-methoxypheLlyl]pyrimidin-2-yl}-2-oxo-2,3-dihydro-1*H*-benzimidazole-1-carboxylate (Compound No. 24)

**Step a: [4-(3-Cyclopentyloxy-4-methoxyphenyl)-pyrimidin-2-yl]-(2-nitrophenyl)-amine**

Sodium hydride (2 eq.) was added to a solution of 4-[3-(cyclopentyloxy)-4-methoxyphenyl]pyrimidin-2-amine (3.5 mmol), cooled at 0-5°C, in dry tetrahydrofuran and the mixture was stirred at same temperature for 30 minutes. *o*-Fluoronitrobenzene (1.5 eq.) was added and the resulting mixture was allowed to come to room temperature. The reaction mixture was heated at reflux for 12 hours. The organic solvent was distilled off and crude mass was dissolved in water and extracted with ethyl acetate, washed with brine, dried over anhydrous sodium sulfate and the solvent was evaporated. The residue thus obtained was purified by column chromatography to furnish the title compound.

**Step b: *N*-[4-(3-cyclopentyloxy-4-methoxyphenyl)-pyrimidin-2-yl]-benzene-1,2-diamine**

Palladium on carbon (10% w/w) was added to a solution of the compound obtained from step a above (3.72 mmol) in ethanol (25 mL). The reaction mixture was stirred for 12 hours under $H_2$ gas atmosphere. It was filtered through celite bed and the crude product thus obtained was purified by column chromatography to furnish title compound.

**Step c: *tert*-Butyl 3-{4-[3-(cyclopentyloxy)-4-methoxyphenyl]pyrimidin-2-yl}-2-oxo-2,3-dihydro-1*H*-benzimidazole-1-carboxylate (Compound No. 24)**

Di-*tert*-butyl-di-carbonate (4 eq.) and 4-dimethylaminopyridine (10% w/w) were added to a solution of the compound obtained from step b above (2.12 mmol) in acetonitrile and the reaction mixture was stirred for 2 hours. It was concentrated under reduced pressure and the residue was purified by column chromatography to furnish the title compound. Yield= 61 %.
IR (neat): 2962, 1772, 1736, 1577, 1484, 1323, 1273, 1152 $cm^{-1}$.
$^1$H NMR (300 MHz, $CDCl_3$): δ 8.84 (d, *J*= 5.26 Hz, 1H), 7.85-8.00 (m, 3H), 7.59-7.76 (m, 2H), 7.11-7.33 (m, 3H), 6.96-7.02 (m, 1H), 4.89-4.90 (m, 1H), 3.94 (s, 3H), 1.86-2.04 (m, 8H), 1.69 (s, 9H).
Mass (*m*/*z*) : 502 $(M)^+$.

Following compounds were prepared similarly
*tert*-Butyl 3- {4-[4-(cyclopentyloxy)-3-methoxyphenyl]pyrimidin-2-yl}-2-oxo-2,3-dihydro-1*H*-benzimidazole-1-carboxylate (Compound No. 2)
Mass (*m*/*z*) : 503.1 $(M+1)^+$.
*tert*-Butyl 3- {5-[3-(cyclopentyloxy)-4-methoxyphenyl]pyrimidin-2-yl}-2-oxo-2,3-dihydro-1*H*-benzimidazole-1-carboxylate (Compound No. 3)
Mass (*m*/*z*) : 502.9 $(M+1)^+$.
*tert*-Butyl 3-[5-(3,4-dimethoxyphenyl)pyrimidin-2-yl]-2-oxo-2,3-dihydro-1H benzimidazole-1-carboxylate (Compound No. 4)
Mass (*m*/*z*) : 449.3 $(M+1)^+$.
*tert*-Butyl 3-[5-(2,5-dimethoxyphenyl)pyrimidin-2-yl]-2-oxo-2,3-dihydro-1H benzimidazole-1-carboxylate (Compound No. 5)
Mass (*m*/*z*) : 449.6 $(M+1)^+$.
*tert*-Butyl 3-[4-(3,4-dimethoxyphenyl)pyrimidin-2-yl]-2-oxo-2,3-dihydro-1*H*-benzimidazole-1-carboxylate (Compound No. 6)
Mass (*m*/*z)* : 448.7 $(M+1)^+$.
*tert*-Butyl-3-{5-[3-(cyclopentyloxy)-4-methoxyphenyl]pyridin-2-yl}-2-oxo-2,3-dihydro-1*H*-benzimidazole-1-carboxylate (Compound No. 15)
Mass (*m*/*z*) : 501.7 $(M+1)^+$.

Example 2: Synthesis of 1-[4-(3-cyclopentyloxy-4-methoxyphenyl)-pyrimidin-2-yl]-1,3-dihydro-2H-benzoimidazol-2-one (Compound No. 25)

Anhydrous potassium carbonate (10 mol%) was added to a solution of *tert*-butyl 3-{4-[3-(cyclopentyloxy)-4-methoxyphenyl]pyrimidin-2-yl}-2-oxo-2,3-dihydro-1*H*-benzimidazole-1-carboxylate (Compound No. 24) (1.29 mmol) in methanol and reaction mixture was refluxed for 2 hours. The resulting mixture was filtered and the residue thus obtained was purified by column chromatography to furnish the title compound. Yield = 66%.
IR (KBr): 2960, 1751, 1710, 1633,1578, 1518, 1409, 1273, 1180 $cm^{-1}$.
$^1H$ NMR (300 MHz, $CDCl_3$ + $CD_3OD$ ): δ 8.79 (bs, 1H), 7.96-7.98 (m, 2H), 7.71-7.77 (m, 3H), 7.09-7.14 (m, 3H), 4.94 (bs, 1H), 3.94 (s, 3H), 1.88-1.99 (m, 6H), 1.67 (bs, 2H). Mass (*m*/*z*) : 402 $(M)^+$.
Following compounds were prepared similarly
1-{4-[4-(Cyclopentyloxy)-3-methoxyphenyl]pyrimidin-2-yl}-1,3-dihydro-2*H*-benzimidazol-2-one (Compound No. 1)
Mass (*m*/*z*) : 402.9 $(M+1)^+$.
1-[5-(3,4-Dimethoxyphenyl)pyrimidin-2-yl]-1,3-dihydro-2*H*-benzimidazol-2-one (Compound No. 7)
Mass (*m*/*z*) : 349.59 $(M+1)^+$.
1-[5-(2,5-Dimethoxyphenyl)pyrimidin-2-yl]-1,3-dihydro-2*H*-benzimidazol-2-one (Compound No. 8)
Mass (*m*/*z*) : 349.6 $(M+1)^+$.
1-{5-[3-(Cyclopentyloxy)-4-methoxyphenyl]pyrimidin-2-yl}-1,3-dihydro-2*H*-benzimidazol-2-one (Compound No. 9)
Mass (*m*/*z*) : 403.1 $(M+1)^+$.
1-[4-(3,4-Dimethoxyphenyl)pyrimidin-2-yl]-1,3-dihydro-2*H*-benzimidazol-2-one (Compound No. 10)
Mass (*m*/*z*) : 349.1 $(M+1)^+$.
1-{5-[3-(Cyclopentyloxy)-4-methoxyphenyl]pyridin-2-yl}-1,3-dihydro-2*H*-benzimidazol-2-one (Compound No. 20)
Mass (*m*/*z*) : 401.9 $(M+1)^+$.

Example 3: Synthesis of 1-cyclopentyl-3-[4-(3,4-dimethoxyphenyl)pyrimidin-2-yl]-1,3-dihydro-2*H*-benzimidazol-2-one (Compound No. 13)

Sodium hydride (0.574 mmol) was added to the stirred solution of 1-[4-(3,4-dimethoxyphenyl)pyrimidin-2-yl]-1,3-dihydro-2*H*-benzimidazol-2-one (Compound No. 10) (0.287 mmol) in dimethylformamide (1.5 mL) at 0 °C, followed by the addition of cyclopentyl bromide (0.430 mmol) after a few minutes. The reaction was continued at 90 °C for 6 hours. The resulting mixture was poured in ice-cold water and extracted with ethyl acetate. Organic phase was dried over anhydrous sodium sulphate and evaporated under reduced pressure to get the crude product. The crude product was purified by column chromatography to furnish the title compound. Yield = 37 %.
IR (KBr): 2957, 2927, 1723, 1575, 1517, 1487, 1407, 1275, 1227, 1152, 1024 $cm^{-1}$
$^1H$ NMR (300 MHz; $CDCl_3$): 8.85 (d, *J* = 5.13 Hz, 1H), 8.03 (d, *J* = 7.47 Hz, 1H), 7.85 (s, 1H), 7.76 (d, *J*= 8.28 Hz, 1H), 7.58 (d, *J*= 5.19 Hz, 1H), 6.75-7.20(m, 4H), 4.94-5.00 (m, 1H), 3.99 (s, 3H) 3.97 (s, 3H), 2.04-2.22 (m, 6H), 1.76 (brs, 2H). Mass (*m*/*z*) : 417.1 $(M+1)^+$.
Following compounds were prepared similarly
1-[4-(3, 4-Dimethoxyphenyl)pyrimidin-2-yl]-3-ethyl-1,3-dihydro-2*H*-benzimidazol-2-one (Compound No. 12)
Mass (*m*/*z*) : 376.9 $(M+1)^+$.
1-Benzyl-3-[4-(3, 4-dimethoxyphenyl)pyrimidin-2-yl]-1,3-dihydro-2*H*-benzimidazol-2-one (Compound No. 14)
Mass (*m*/*z*) : 438.9 $(M+1)^+$.
1- {5-[3-(Cyclopentyloxy)-4-methoxyphenyl]pyridin-2-yl}-3-methyl-1,3-dihydro-2*H*-benzimidazol-2-one (Compound No. 16)
Mass (*m*/*z*) : 416.1 $(M+1)^+$.
1-Allyl-3- {5-[3-(cyclopentyloxy)-4-methoxyphenyl]pyridin-2-yl}-1,3-dihydro-2*H*-benzimidazol-2-one (Compound No. 17)
Mass (*m*/*z*) : 442.0 $(M+1)^+$.
1-Benzyl-3- {5-[3-(cyclopentyloxy)-4-methoxyphenyl]pyridin-2-yl}-1,3-dihydro-2*H*-benzimidazol-2-one (Compound No. 18)
Mass (*m*/*z*) : 492.0 $(M+1)^+$.
1-{5-[3-(Cyclopentyloxy)-4-methoxyphenyl]pyridin-2-yl}-3-ethyl-1,3-dihydro-2*H*-benzimidazol-2-one (Compound No. 19)
Mass (*m*/*z*) : 430.2 $(M+1)^+$.
1-{5-[3-(Cyclopentyloxy)-4-methoxyphenyl]pyridin-2-yl}-3-propyl-1,3-dihydro-2*H*-benzimidazol-2-one (Compound No. 21)
Mass (*m*/*z*) : 443.2 $(M+1)^+$.
1-Cyclopentyl-3- {5-[3-(cyclopentyloxy)-4-methoxyphenyl]pyridin-2-yl}-1,3-dihydro-2*H*-benzimidazol-2-one (Compound No. 22)

Mass ($m/z$) : 470.3 $(M+1)^+$.
1-{5-[3-(Cyclopentyloxy)-4-methoxyphenyl]pyridin-2-yl}-3-isopropyl-1,3-dihydro-2*H*-benzimidazol-2-one (Compound No. 23)
Mass ($m/z$) : 444.1 $(M+1)^+$.
1-{5-[3-(Cyclopentyloxy)-4-methoxyphenyl]pyridin-2-yl}-3-(4-methoxybenzyl)-1,3-dihydro-2*H*-benzimidazol-2-one (Compound No. 30)
Mass ($m/z$) : 522.9 $(M+1)^+$.
1-{5-[3-(Cyclopentyloxy)-4-methoxyphenyl]pyridin-2-yl}-3-[4-(trifluoromethyl)benzyl]-1,3-dihydro-2*H*-benzimidazol-2-one (Compound No. 31)
Mass ($m/z$) : 492 (a fragment peak).
1-{5-[3-(Cyclopentyloxy)-4-methoxyphenyl]pyridin-2-yl}-3-(pyridin-3-ylmethyl)-1,3-dihydro-2*H*-benzimidazol-2-one (Compound No. 32)
Mass ($m/z$) : 493.2 $(M+1)^+$.
1-{5-[3-(Cyclopentyloxy)-4-methoxyphenyl]pyridin-2-yl}-3-[4-(methylsulfanyl)benzyl]-1,3-dihydro-2*H*-benzimidazol-2-one (Compound No. 44)
Mass ($m/z$) : 538.2 $(M+1)^+$.

Example 4: Synthesis of 1-(difluoromethyl)-3-[4-(3,4-dimethoxyphenyl)pyrimidin-2-yl]-1,3-dihydro-2*H*-benzimidazol-2-one (Compound No. 11)

Cesium carbonate (0.577 mmol) was added to a stirred solution of 1-[4-(3,4-dimethoxyphenyl)pyrimidin-2-yl]-1,3-dihydro-2*H*-benzimidazol-2-one (Compound No. 10) (0.481mmol) in dry dimethylformamide (1.5 mL) and the reaction mixture was stirred for 30 minutes. Methyl-2-chloro-2,2-difluoroacetate (0.48 mol) was added to reaction mixture and reaction was continued at 80 °C for 4 h. The reaction mixture was poured on ice water and extracted with ethyl acetate. Organic phase was dried over anhydrous sodium sulphate and evaporated under reduced pressure. Crude product was purified by column chromatography to yield titled compound as white solid. Yield =24 %.
IR (KBr): 2926, 1769, 1577, 1545, 1488, 1405, 1377, 1309, 1277, 1181, 1156, 1051, 1027 775 $cm^{-1}$
$^1H$ NMR (300 MHz; $CDCl_3$): 8.85 (d, $J$= 5.07 Hz, 1H), 8.03 (d, $J$= 6.36 Hz, 1H), 7.84 (s, 1H), 7.76 (d, $J$= 8.01 Hz, 1H), 7.64 (d, $J$= 5.37 Hz, 2H), 7.46 (s, 2H), 7.01 (d, $J$= 8.16 Hz, 1H), 3.99 (s, 3H), 3.98 (s, 3H)
Mass ($m/z$): 399.1 $[M+1]^+$

Example 5: Synthesis of 3-{5-[3-(cyclopentyloxy)-4-methoxphenyl]byrimidin-2-yl}-*N*-isopropyl-2-oxo-2,3-dihydro-1*H*-benzimidazole-1-carboxamide (Compound No. 26)

Isopropyl isocyanate (1.2 mmol) was added to a solution 1-{5-[3-(cyclopentyloxy)-4-methoxyphenyl]pyrimidin-2-yl}-1,3-dihydro-2*H*-benzimidazol-2-one (Compound No. 9) (0.4 mmol) and 4-dimethylaminopyridine (0.1 mmol) in dry dichloromethane. The reaction mixture was refluxed for 12 hours, diluted with dichloromethane and washed with water, brine, dried over sodium sulphate and the solvent was evaporated. The mixture was concentrated under reduced pressure and the residue thus obtained was purified by column chromatography to furnish the title compound. Yield = 30%.
IR ($CHCl_3$): 3313, 2963, 2929, 1741, 1702, 1586, 1537, 1478, 1434, 1386, 1318, 1257, 1177, 1144, 1102, 973 $cm^{-1}$
$^1H$ NMR ($CDCl_3$, 300 MHz) $\delta$: 9.04 (s, 2H), 8.74 (d, $J$ = 7.19 Hz, 1H), 8.35-8.38 (m, 1H), 7.90-7.93 (m, 1H), 7.27-7.31 (m, 1H), 7.15-7.16 (m, 1H), 7.11 (s, 1H), 7.03 (d, $J$ = 8.29 Hz, 1H), 4.85-4.88 (m, 1H), 4.14-4.21 (m, 1H), 3.92 (s, 3H), 1.81-1.99 (m, 6H), 1.64-1.68 (m, 2H), 1.30 (d, $J$= 6.57 Hz, 6H).
Mass ($m/z$): 487.9 $(M+1)^+$.
Following compounds were prepared similarly
3-{4-[3-(Cyclopentyloxy)-4-methoxyphenyl]pyrimidin-2-yl}-*N*-isopropyl-2-oxo-2,3-dihydro-1*H*-benzimidazole-1-carboxamide (Compound No. 27)
Mass ($m/z$) : 488.0 $(M+1)^+$.
*N*-Cyclopentyl-3- {5-[3-(cyclopentyloxy)-4-methoxyphenyl]pyrimidin-2-yl}-2-oxo-2,3-dihydro-1*H*-benzimidazole-1-carboxamide (Compound No. 28)
Mass ($m/z$) : 513.9 $(M+1)^+$.
*N*-(2-chloroethyl)-3- {5-[3-(cyclopentyloxy)-4-methoxyphenyl]pyridin-2-yl}-2-oxo-2,3-dihydro-1*H*-benzimidazole-1-carboxamide (Compound No. 45)
Mass ($m/z$) : 507.0 $(M+1)^+$.
*N*-(3-chloropropyl)-3-{5-[3-(cyclopentyloxy)-4-methoxyphenyl]pyridin-2-yl}-2-oxo-2,3-dihydro-1*H*-benzimidazole-1-carboxamide (Compound No. 46)
Mass ($m/z$) : 520.9 $(M+1)^+$.
3-{5-[3-(Cyclopentyloxy)-4-methoxyphenyl]pyridin-2-yl}-2-oxo-*N*-prop-2-en-1-yl-2,3-dihydro-1*H*-benzimidazole-1-carboxamide (Compound No. 47)
Mass (*m/z*) : 484.53 $(M)^+$.
*N*-benzyl-3-{5-[3-(cyclopentyloxy)-4-methoxyphenyl]pyridin-2-yl}-2-oxo-2,3-dihydro-1*H*-benzimidazole-1-carboxamide (Compound No. 48)
Mass (*m/z*) : 534.4 $(M)^+$.
3-{5-[3-(Cyclopentyloxy)-4-methoxyphenyl]pyridin-2-yl}-2-oxo-*N*-pentyl-2,3-dihydro-1*H*-benzimidazole-1-carboxamide (Compound No. 49)
Mass (*m/z*) : 514.5 $(M)^+$.
*N*-cyclopentyl-3-{5-[3-(cyclopentyloxy)-4-methoxyphenyl]pyridin-2-yl}-2-oxo-2,3-dihydro-1*H*-benzimidazole-1-carboxamide (Compound No. 50)
Mass (*m/z*) : 512.4 $(M)^+$.
3-{5-[3-(Cyclopentyloxy)-4-methoxyphenyl]pyridin-2-yl}-*N*-heptyl-2-oxo-2,3-dihydro-1*H*-benzimidazole-1-carboxamide (Compound No. 51)
Mass (*m/z*) : 542.4 $(M)^+$.
3-{5-[3-(Cyclopentyloxy)-4-methoxyphenyl]pyridin-2-yl}-*N*-(4-methylphenyl)-2-oxo-2,3-dihydro-1*H*-benzimidazole-1-carboxamide (Compound No. 52)
Mass (*m/z*) : 534.4 $(M)^+$.
*N*-(4-bromophenyl)-3-{5-[3-(cyclopentyloxy)-4-methoxyphenyl]pyridin-2-yl}-2-oxo-2,3-dihydro-1*H*-benzimidazole-1-carboxamide (Compound No. 53)
Mass (*m/z*) : 598 $(M)^+$.

Example 6: Synthesis of 1-cyclopentyl-3-{5-[3-(cyclopentyloxy)-4-methoxyphenyl]pyrimidin-2-yl}-1,3-dihydro-2*H*-benzimidazol-2-one (Compound No. 29)

Cyclopentyl bromide (1.1 equivalent) was added to a mixture of 1-{5-[3-(cyclopentyloxy)-4-methoxyphenyl] pyrimidin-2-yl}-1,3-dihydro-2*H*-benzimidazol-2-one (Compound No. 9) (0.4 mmol) and potassium *tert*-butoxide (0.4 mmol) in acetonitrile. The reaction mixture was stirred at reflux temperature. After 12 hours, reaction mixture was diluted with dichloromethane and washed with water, brine, dried over anhydrous sodium sulphate and evaporated at reduced pressure to give a residue, which was purified by eluting through silica gel column to give white solid compound. Yield = 40%
$^1$H NMR (300 MHz; $CDCl_3$): 1.76-2.22 (m, 16H), 3.91 (s, 6H), 4.87- 4.98 (m, 2H), 6.99-7.16 (m, 6H), 7.95 (d, *J*= 8.26 Hz, 1H), 9.01 (s, 2H)
Mass (*m/z*): 471 $(M+1)^+$.
Following compound was prepared similarly
1-Cyclopentyl-3- {4-[3-(cyclopentyloxy)-4-methoxyphenyl]pyrimidin-2-yl}-1,3-dihydro-2*H*-benzimidazol-2-one (Compound No. 43)
Mass (*m/z*): 470 $(M)^+$.

Example 7: Synthesis of 1-acetyl-3-{5-[3-(cyclopentyloxy)-4-methoxyphenyl]pyrimidin-2-yl}-1,3-dihydro-2*H*-benzimidazol-2-one (Compound No. 33)

Acetic anhydride (1.1 equivalent) was added to a mixture of 1-{5-[3-(cyclopentyloxy)-4-methoxyphenyl]pyrimidin-2-yl}-1,3-dihydro-2*H*-benzimidazol-2-one (Compound No. 9) (0.4 mmol) and potassium *tert*-butoxide (0.4 mmol) in *tert*-butanol. The reaction mixture was stirred at room temperature. After 2 hours, reaction mixture was diluted with dichloromethane and washed with water, brine, dried over anhydrous sodium sulphate and evaporated at reduced pressure to give a residue, which was purified by eluting through silica gel column to give white solid compound. Yield = 30%
IR (KBr):2965, 1769, 1712, 1598, 1516, 1479, 1434, 1418, 1374, 1313, 1260, 1161, 1017 $cm^{-1}$
$^1$H NMR ($CDCl_3$, 300 MHz) δ: 9.05 (s, 2H), 8.31-8.33 (m, 1H), 7.85- 7.87 (m, 2H), 7.19-7.28 (m, 2H), 7.16- 7.19 (m, 1H), 7.02- 7.11 (m, 2H), 4.88 (m, 1H), 3.93 (s. 3H), 2.86 (s, 3H), 1.89-1.97 (m, 6H), 1.57-1.66 (m, 2H)
Mass (*m/z*): 445.1 $(M+1)^+$.
Following compounds were prepared similarly
1- {5-[3-(Cyclopentyloxy)-4-methoxyphenyl]pyrimidin-2-yl}-3-propanoyl-1,3-dihydro-2*H*-benzimidazol-2-one (Compound No. 34)
Mass (*m/z*) : 459.0 $(M+1)^+$.
1-Butanoyl-3-{5-[3-(cyclopentyloxy)-4-methoxyphenyl]pyrimidin-2-yl}-1,3-dihydro-2*H*-benzimidazol-2-one (Compound No. 35)
Mass (*m/z*) : 472.9 $(M+1)^+$.

1-{5-[3-(Cyclopentyloxy)-4-methoxyphenyl]pyrimidin-2-yl}-3-(2-methylpropanoyl)-1,3-dihydro-2*H*-benzimidazol-2-one (Compound No. 36)
Mass (*m*/*z*) : 472.9 $(M+1)^+$.
1-{5-[3-(Cyclopentyloxy)-4-methoxyphenyl]pyrimidin-2-yl}-3-(phenylcarbonyl)-1,3-dihydro-2*H*-benzimidazol-2-one (Compound No. 37)
Mass (*m*/*z*) : 507.1 $(M+1)^+$.
1-{5-[3-(Cyclopentyloxy)-4-methoxyphenyl]pyrimidin-2-yl}-3-(2,2-dimethylpropanoyl)-1,3-dihydro-2*H*-benzimidazol-2-one (Compound No. 38)
Mass (*m*/*z*) : 487.1 $(M+1)^+$.
1-{5-[3-(Cyclopentyloxy)-4-methoxyphenyl]pyridin-2-yl}-3-(phenylcarbonyl)-1,3-dihydro-2*H*-benzimidazol-2-one (Compound No. 39)
Mass (*m*/*z*) : 506.1 $(M+1)^+$.
1-{5-[3-(Cyclopentyloxy)-4-methoxyphenyl]pyridin-2-yl}-3-propanoyl-1,3-dihydro-2*H*-benzimidazol-2-one (Compound No. 40)
Mass (*m*/*z*) : 458.1 $(M+1)^+$.
1-Butanoyl-3-{5-[3-(cyclopentyloxy)-4-methoxyphenyl]pyridin-2-yl}-1,3-dihydro-2*H*-benzimidazol-2-one (Compound No. 41)
Mass (*m*/*z*) : 472 $(M+1)^+$.
1-Acetyl-3-{5-[3-(cyclopentyloxy)-4-methoxyphenyl]pyridin-2-yl}-1,3-dihydro-2*H*-benzimidazol-2-one (Compound No. 42)
Mass (*m*/*z*) : 444.1 $(M+1)^+$.

Example 8: Biological Assay

PDE 4 Enzyme Assay

The efficacy of particular compounds specifically disclosed herein was determined by an enzyme assay using cell lysate of HEK293 cells transfected with PDE4B2 plasmids as PDE4B source. The enzyme reaction was carried out in the presence of cAMP (1 μM) at 30°C in the presence or absence of test compound for 45 -60 min. An aliquot of this reaction mixture was taken further for the ELISA assay and the protocol of the kit followed to determine level of cAMP in the sample. The concentration of the cAMP in the sample directly correlated with the degree of PDE 4 enzyme inhibition. Results were expressed as percent control and the $IC_{50}$ values of test compounds were reported. $IC_{50}$ values of test compounds were found to be in the range of μM to nM concentration. Compound Nos. 3-43 and 47-53 displayed $IC_{50}$ for the PDE 4 enzyme assay ranging from about 140 nM to about 10 μM, for example, from about 140 nM to about 1 μM, or from about 140 nM to about 500 nM.

Cell based Assay for TNF-α release

*Method of isolation of Human Peripheral Blood Mononuclear Cells (PBMNCs)*

Human whole blood is collected in vacutainer tubes containing heparin or EDTA as an anti coagulant. The blood is diluted (1:1) in sterile phosphate buffered saline and 10 ml is carefully layered over 5 ml Ficoll Hypaque gradient (density 1.077 g/ml) in a 15 ml conical centrifuge tube. The sample is centrifuged at 3000 rpm for 25 minutes in a swing-out rotor at room temperature. After centrifugation, interface of cells are collected, diluted at least 1:5 with phosphate-buffered saline (PBS) and washed three times by centrifugation at 2500 rpm for 10 minutes at room temperature. The cells are resuspended in serum free RPMI 1640 medium at a concentration of 2 million cells/ml.

*LPS (Lipopolysaccharide) stimulation of Human PBMNCs*

PBMN cells (0.1 ml; 2 million/ml) are co-incubated with 20 ml of compound (final DMSO concentration of 0.2 %) for 10 min in a flat bottom 96 well microtiter plate. Compounds are dissolved in DMSO initially and diluted in medium for a final concentration of 0.2 % DMSO. LPS (1 mg/ml, final concentration) is then added at a volume of 10 μl per well. After 30 min, 20 μl of fetal calf serum (final concentration of 10 %) is added to each well. Cultures are incubated overnight at 37 °C in an atmosphere of 5 % $CO_2$ and 95 % air. Supernatant are then removed and tested by ELISA for TNF-α release using a commercial kit (e.g. BD Biosciences). The level of TNF-α in treated wells is compared with the vehicle treated controls and inhibitory potency of compound is expressed as $IC_{50}$ values calculated by using Graph pad prism.

$$\text{Percent inhibition} = 100 - \frac{\text{Percent TNF-}\alpha\text{ drug treated}}{\text{Percent TNF-}\alpha\text{ in vehicle treated}} \times 100$$

## Claims

1. A compound having the structure of Formula I,

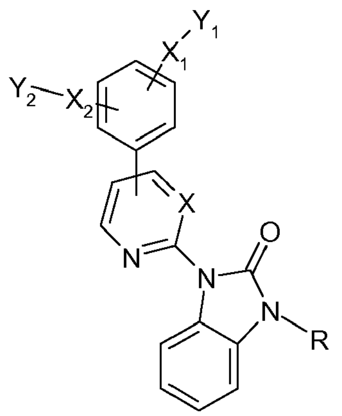

**Formula I**

and its pharmaceutically acceptable salts, pharmaceutically acceptable solvates, enantiomers, diastereomers or N-oxides, wherein
$Y_1$ and $Y_2$ independently are hydrogen, alkyl, cycloalkyl, heterocyclyl, heteroaryl, aralkyl, heterocyclylalkyl or heteroarylalkyl;
$X_1$ and $X_2$ independently are oxygen or sulphur;
X is nitrogen or carbon;
R is hydrogen, alkyl, alkenyl, alkynyl, cycloalkyl, aryl, aralkyl, heteroaryl, heterocyclyl, heteroarylalkyl, heterocyclylalkyl, $-COR_x$, $-NR_xR_y$, carboxy, or $-C(=X_3)NR_xR_y$;
$R_x$ and $R_y$ independently are hydrogen, alkyl, alkenyl, cycloalkyl, aryl, heterocyclyl, heteroaryl, aralkyl, heterocyclylalkyl or heteroarylalkyl;
$R_x$ and $R_y$ optionally join together to form cycloalkyl or heterocyclyl ring and $X_3$ can be oxygen or sulfur.

2. A compound which is selected from:

1- {4-[4-(Cyclopentyloxy)-3-methoxyphenyl]pyrimidin-2-yl}-1,3-dihydro-2*H*-benzimidazol-2-one,
*tert*-Butyl 3- {4-[4-(cyclopentyloxy)-3-methoxyphenyl]pyrimidin-2-yl}-2-oxo-2,3-dihydro-1*H*-benzimidazole-1-carboxylate,
*tert*-Butyl 3- {5-[3-(cyclopentyloxy)-4-methoxyphenyl]pyrimidin-2-yl}-2-oxo-2,3-dihydro-1*H*-benzimidazole-1-carboxylate,
*tert*-Butyl 3-[5-(3,4-dimethoxyphenyl)pyrimidin-2-yl]-2-oxo-2,3-dihydro-1*H*-benzimidazole-1-carboxylate,
*tert*-Butyl 3-[5-(2,5-dimethoxyphenyl)pyrimidin-2-yl]-2-oxo-2,3-dihydro-1*H*-benzimidazole-1-carboxylate,
*tert*-Butyl 3-[4-(3,4-dimethoxyphenyl)pyrimidin-2-yl]-2-oxo-2,3-dihydro-1*H*-benzimidazole-1-carboxylate,
1-[5-(3,4-Dimethoxyphenyl)pyrimidm-2-yl]-1,3-dihydro-2*H*-benzimidazo1-2-one,
1-[5-(2,5-Dimethoxyphenyl)pyrimidin-2-yl]-1,3-dihydro-2*H*-benzimidazo1-2-one,
1-{5-[3-(Cyclopentyloxy)-4-methoxyphenyl]pyrimidm-2-yl}-1,3-dihydro-2*H*-benzimidazol-2-one,
1-[4-(3,4-Dimethoxyphenyl)pyrimidin-2-yl]-1,3-dihydro-2*H*-benzimidazo1-2-one,
1-(Difluoromethyl)-3-[4-(3,4-dimethoxyphenyl)pyrimidin-2-yl]-1,3-dihydro-2*H*-benzimidazol-2-one,

1-[4-(3,4-Dimethoxyphenyl)pyrimidin-2-yl]-3-ethyl-1,3-dihydro-2*H*-benzimidazol-2-one,
1-Cyclopentyl-3-[4-(3,4-dimethoxyphenyl)pyrimidin-2-yl]-1,3-dihydro-2*H*-benzimidazol-2-one,
1-Benzyl-3-[4-(3, 4-dimethoxyphenyl)pyrimidin-2-yl]-1,3-dihydro-2*H*-benzimidazol-2-one,
*tert*-Butyl-3-{5-[3-(cyclopentyloxy)-4-methoxyphenyl]pyridin-2-yl}-2-oxo-2,3-dihydro-1*H*-benzimidazole-1-carboxylate,
1-{5-[3-(Cyclopentyloxy)-4-methoxyphenyl]pyridin-2-yl}-3-methyl-1,3-dihydro-2*H*-benzimidazol-2-one,
1-Allyl-3- {5-[3-(cyclopentyloxy)-4-methoxyphenyl]pyridin-2-yl}-1,3-dihydro-2*H*-benzimidazol-2-one,
1-Benzyl-3- {5-[3-(cyclopentyloxy)-4-methoxyphenyl]pyridin-2-yl}-1,3-dihydro-2*H*-benzimidazol-2-one,
1-{5-[3-(Cyclopentyloxy)-4-methoxyphenyl]pyridin-2-yl}-3-ethyl-1,3-dihydro-2*H*-benzimidazol-2-one,
1-{5-[3-(Cyclopentyloxy)-4-methoxyphenyl]pyridin-2-yl}-1,3-dihydro-2*H*-benzimidazol-2-one,
1-{5-[3-(Cyclopentyloxy)-4-methoxyphenyl]pyridin-2-yl}-3-propyl-1,3-dihydro-2*H*-benzimidazol-2-one,
1-Cyclopentyl-3- {5-[3-(cyclopentyloxy)-4-methoxyphenyl]pyridin-2-yl}-1,3-dihydro-2*H*-benzimidazol-2-one,
1-{5-[3-(Cyclopentyloxy)-4-methoxyphenyl]pyridm-2-yl}-3-isopropyl-1,3-dihydro-2*H*-benzimidazol-2-one,
*tert*-Butyl-3-{4-[3-(cyclopentyloxy)-4-methoxyphenyl]pyrimidin-2-yl}-2-oxo-2,3-dihydro-1*H*-benzimidazole-1-carboxylate,
1-{4-[3-(Cyclopentyloxy)-4-methoxyphenyl]pyrimidm-2-yl}-1,3-dihydro-2*H*-benzimidazol-2-one,
3-{5-[3-(Cyclopentyloxy)-4-methoxyphenyl]pyrimidin-2-yl}-*N*-isopropyl-2-oxo-2,3-dihydro-1*H*-benzimidazole-1-carboxamide,
3-{4-[3-(Cyclopentyloxy)-4-methoxyphenyl]pyrimidin-2-yl}-*N*-isopropyl-2-oxo-2,3-dihydro-1*H*-benzimidazole-1-carboxamide,
*N*-Cyclopentyl-3-{5-[3-(cyclopentyloxy)-4-methoxyphenyl]pyrimidin-2-yl}-2-oxo-2,3-dihydro-1*H*-benzimidazole-1-carboxamide,
1-Cyclopentyl-3- {5-[3-(cyclopentyloxy)-4-methoxyphenyl]pyrimidm-2-yl}-1,3-dihydro-2*H*-benzimidazol-2-one,
1-{5-[3-(Cyclopentyloxy)-4-methoxyphenyl]pyridin-2-yl}-3-(4-methoxybenzyl)-1,3-dihydro-2*H*-benzimidazol-2-one,
1-{5-[3-(Cyclopentyloxy)-4-methoxyphenyl]pyridin-2-yl}-3-[4-(trifluoromethyl)benzyl]-1,3-dihydro-2*H*-benzimidazol-2-one,
1-{5-[3-(Cyclopentyloxy)-4-methoxyphenyl]pyridin-2-yl}-3-(pyridin-3-ylmethyl)-1,3-dihydro-2*H*-benzimidazol-2-one,
1-Acetyl-3-{5-[3-(cyclopentyloxy)-4-methoxyphenyl]pyrimidin-2-yl}-1,3-dihydro-2*H*-benzimidazol-2-one,
1-{5-[3-(Cyclopentyloxy)-4-methoxyphenyl]pyrimidin-2-yl}-3-propanoyl-1,3-dihydro-2*H*-benzimidazol-2-one,
1-Butanoyl-3- {5-[3-(cyclopentyloxy)-4-methoxyphenyl]pyrimidin-2-yl}-1,3-dihydro-2*H*-benzimidazol-2-one,
1-{5-[3-(Cyclopentyloxy)-4-methoxyphenyl]pyrimidin-2-yl}-3-(2-methylpropanoyl)-1,3-dihydro-2*H*-benzimidazol-2-one,
1-{5-[3-(Cyclopentyloxy)-4-methoxyphenyl]pyrimidin-2-yl}-3-(phenylcarbonyl)-1,3-dihydro-2*H*-benzimidazol-2-one,
1-{5-[3-(Cyclopentyloxy)-4-methoxyphenyl]pyrimidin-2-yl}-3-(2,2-dimethylpropanoyl)-1,3-dihydro-2*H*-benzimidazol-2-one,
1-{5-[3-(Cyclopentyloxy)-4-methoxyphenyl]pyridm-2-yl}-3-(phenylcarbonyl)-1,3-dihydro-2*H*-benzimidazol-2-one,
1-{5-[3-(Cyclopentyloxy)-4-methoxyphenyl]pyridin-2-yl}-3-propanoyl-1,3-dihydro-2*H*-benzimidazol-2-one,
1-Butanoyl-3- {5-[3-(cyclopentyloxy)-4-methoxyphenyl]pyridin-2-yl}-1,3-dihydro-2*H*-benzimidazol-2-one,
1-Acetyl-3- {5-[3-(cyclopentyloxy)-4-methoxyphenyl]pyridm-2-yl}-1,3-dihydro-2*H*-benzimidazol-2-one,
1-Cyclopentyl-3- {4-[3-(cyclopentyloxy)-4-methoxyphenyl]pyrimidm-2-yl}-1,3-dihydro-2*H*-benzimidazol-2-one,
1-{5-[3-(Cyclopentyloxy)-4-methoxyphenyl]pyridin-2-yl}-3-[4-(methylsulfanyl)benzyl]-1,3-dihydro-2*H*-benzimidazol-2-one,
*N*-(2-chloroethyl)-3-{5-[3-(cyclopentyloxy)-4-methoxyphenyl]pyridin-2-yl}-2-oxo-2,3-dihydro-1*H*-benzimidazole-1-carboxamide,
*N*-(3-chloropropyl)-3-{5-[3-(cyclopentyloxy)-4-methoxyphenyl]pyridin-2-yl}-2-oxo-2,3-dihydro-1*H*-benzimidazole-1-carboxamide,
3- {5-[3-(Cyclopentyloxy)-4-methoxyphenyl]pyridin-2-yl}-2-oxo-*N*-prop-2-en-1-yl-2,3-dihydro-1*H*-benzimidazole-1-carboxamide,
*N*-benzyl-3-{5-[3-(cyclopentyloxy)-4-methoxyphenyl]pyridin-2-yl}-2-oxo-2,3-dihydro-1*H*-benzimidazole-1-carboxamide,
3-{5-[3-(Cyclopentyloxy)-4-methoxyphenyl]pyridin-2-yl}-2-oxo-*N*-pentyl-2,3-dihydro-1*H*-benzimidazole-1-carboxamide,
*N*-cyclopentyl-3-{5-[3-(cyclopentyloxy)-4-methoxyphenyl]pyridin-2-yl}-2-oxo-2,3-dihydro-1*H*-benzimidazole-1-carboxamide,

3-{5-[3-(Cyclopentyloxy)-4-methoxyphenyl]pyridin-2-yl}-*N*-heptyl-2-oxo-2,3-dihydro-1*H*-benzimidazole-1-carboxamide,
3-{5-[3-(Cyclopentyloxy)-4-methoxyphenyl]pyridin-2-yl}-*N*-(4-methylphenyl)-2-oxo-2,3-dihydro-1*H*-benzimidazole-1-carboxamide,
*N*-(4-bromophenyl)-3-{5-[3-(cyclopentyloxy)-4-methoxyphenyl]pyridin-2-yl}-2-oxo-2,3-dihydro-1*H*-benzimidazole- 1- carboxamide,

and pharmaceutically acceptable salts, pharmaceutically acceptable solvates, enantiomers, diastereomers or N-oxides.

**3.** A pharmaceutical composition comprising a therapeutically effective amount of a compound of claim 1, along with at least one pharmaceutically acceptable carrier, excipient or diluent.

**4.** A pharmaceutical composition comprising a therapeutically effective amount of a compound of claim 1, along with at least one pharmaceutically acceptable carrier, excipient or diluent and at least one other compound selected from corticosteroids, β2-agonists, leukotriene antagonists, 5-lipoxygenase inhibitors, chemokine inhibitors, muscarinic receptor antagonists, $p^{38}$ MAP kinase inhibitors, anticholinergics, antiallergics, PAF antagonists, EGFR kinase inhibitors, additional PDE 4 inhibitors, or combination(s) thereof.

**5.** A method for treating, preventing, inhibiting or suppressing an inflammatory condition or disease, in a mammal, comprising administering a therapeutically effective amount of a compound of claim 1.

**6.** A method for treating, preventing, inhibiting or suppressing an inflammatory condition or disease, in a mammal, comprising administering a therapeutically effective amount of a pharmaceutical composition of claim 3.

**7.** A method for the treatment, prevention, inhibition or suppression of asthma, arthritis, bronchitis, chronic obstructive pulmonary disease (COPD), psoriasis, allergic rhinitis, shock, atopic dermatitis, Crohn's disease, adult respiratory distress syndrome (ARDS), AIDS, eosinophilic granuloma, allergic conjunctivitis, osteoarthritis, ulcerative colitis or other inflammatory diseases in a mammal comprising administering a therapeutically effective amount of a compound of claim 1.

**8.** A method for the treatment, prevention, inhibition or suppression of asthma, arthritis, bronchitis, chronic obstructive pulmonary disease (COPD), psoriasis, allergic rhinitis, shock, atopic dermatitis, Crohn's disease, adult respiratory distress syndrome (ARDS), AIDS, eosinophilic granuloma, allergic conjunctivitis, osteoarthritis, ulcerative colitis or other inflammatory diseases in a mammal comprising administering a therapeutically effective amount of a pharmaceutical composition of claim 3.

**9.** A method for the preparation of a compound of Formula V,

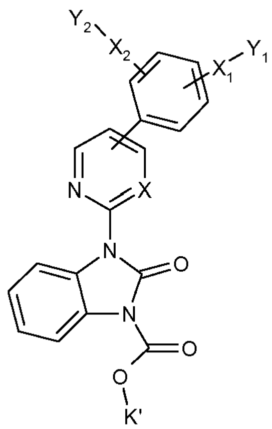

Formula V

the method comprising

a. coupling of a compound of Formula II with a compound of Formula IIa to give a compound of Formula III,

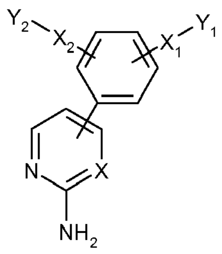
Formula II

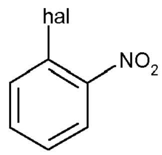
Formula IIa

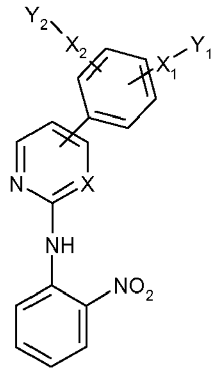
Formula III

b. carrying out reduction of the compound of Formula III to give a compound of Formula IV, and

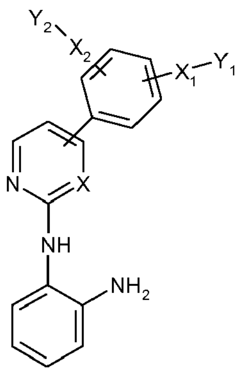
Formula IV

c. cyclizing the compound of Formula IV by reacting with a compound of Formula K to give a compound of Formula V,

wherein $Y_1$ and $Y_2$ independently are hydrogen, alkyl, cycloalkyl, heterocyclyl, heteroaryl, aralkyl, heterocyclylalkyl or heteroarylalkyl;
$X_1$ and $X_2$ independently are oxygen or sulphur;
X is nitrogen or carbon;
hal is fluorine, chlorine, bromine or iodine,
K is $(CO_2CH_2C_6H_5)_2O$, $(CO_2C(CH_3)_3)_2O$, $(CO_2C(CH_3)_2CHBr_2)_2O$ or $(CO_2C(CH_3)_2CCl_3)_2O$ and K' is $-CH_2C_6H_5$, $-C(CH_3)_3$, $-C(CH_3)_2CHBr_2$ or $-C(CH_3)_2CCl_3$.

**10.** A method for the preparation of a compound of Formula VI,

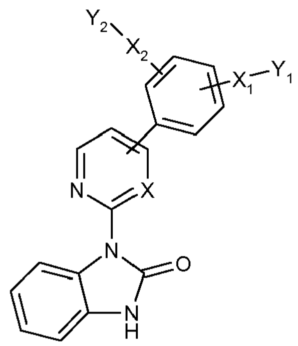

Formula VI

the method comprising

a. coupling of a compound of Formula II with a compound of Formula IIa to give a compound of Formula III,

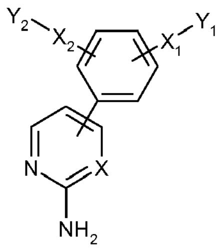

Formula II

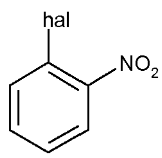

Formula IIa

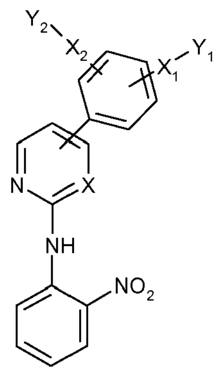

Formula III

b. carrying out reduction of the compound of Formula III to give a compound of Formula IV,

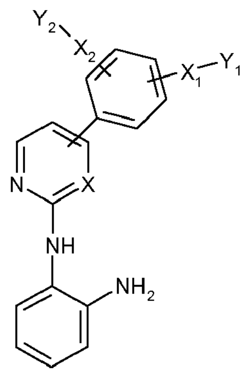

Formula IV

c. cyclizing the compound of Formula IV by reacting with a compound of Formula K to give a compound of Formula V, and

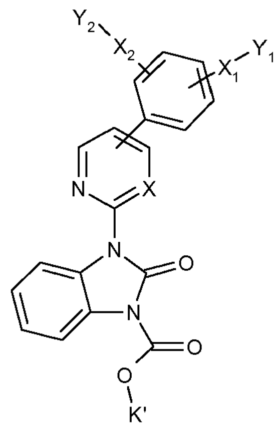

Formula V

d. deprotecting the compound of Formula V to give a compound of Formula VI,

wherein $Y_1$ and $Y_2$ independently are hydrogen, alkyl, cycloalkyl, heterocyclyl, heteroaryl, aralkyl, heterocyclylalkyl or heteroarylalkyl;
$X_1$ and $X_2$ independently are oxygen or sulphur;
X is nitrogen or carbon;
hal is fluorine, chlorine, bromine or iodine,
K is $(CO_2CH_2C_6H_5)_2O$, $(CO_2C(CH_3)_3)_2O$, $(CO_2C(CH_3)_2CHBr_2)_2O$ or $(CO_2C(CH_3)_2CCl_3)_2O$ and K' is $-CH_2C_6H_5$, $-C(CH_3)_3$, $-C(CH_3)_2CHBr_2$ or $-C(CH_3)_2CCl_3$.

**11.** A method for the preparation of a compound of Formula VIII,

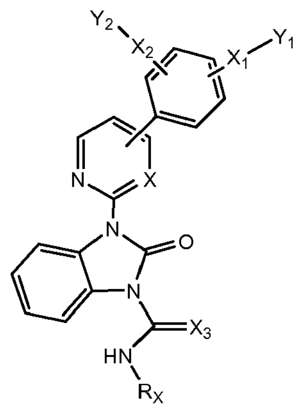

Formula VIII

the method comprising

a. coupling of a compound of Formula II with a compound of Formula IIa to give a compound of Formula III,

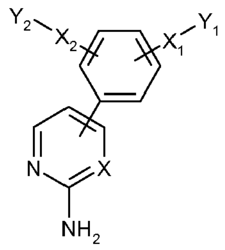
Formula II

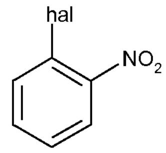
Formula IIa

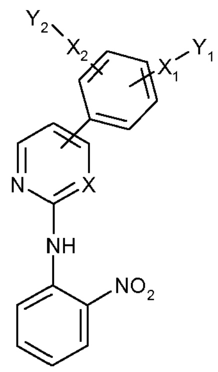
Formula III

b. carrying out reduction of the compound of Formula III to give a compound of Formula IV,

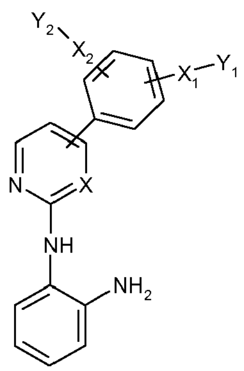
Formula IV

c. cyclizing the compound of Formula IV by reacting with a compound of Formula K to give a compound of Formula V,

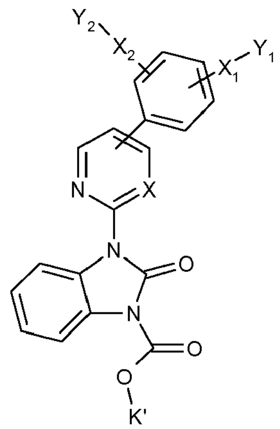
Formula V

d. deprotecting the compound of Formula V to give a compound of Formula VI, and

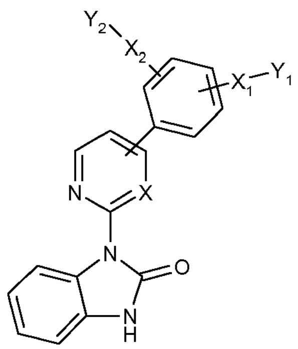

Formula VI

e. reacting the compound of Formula VI with a compound of Formula VII

$R_xN{=}C({=}X_1)$ Formula VII

to give a compound of Formula VIII.
wherein $Y_1$ and $Y_2$ independently are hydrogen, alkyl, cycloalkyl, heterocyclyl, heteroaryl, aralkyl, heterocyclylalkyl or heteroarylalkyl;
$X_1$, $X_2$ and $X_3$ independently are oxygen or sulphur;
X is nitrogen or carbon;
hal is fluorine, chlorine, bromine or iodine,
K is $(CO_2CH_2C_6H_5)_2O$, $(CO_2C(CH_3)_3)_2O$, $(CO_2C(CH_3)_2CHBr_2)_2O$ or $(CO_2C(CH_3)_2CCl_3)_2O$,
K' is $-CH_2C_6H_5$, $-C(CH_3)_3$, $-C(CH_3)_2CHBr_2$ or $-C(CH_3)_2CCl_3$, and
$R_x$ is hydrogen, alkyl, alkenyl, cycloalkyl, aryl, heterocyclyl, heteroaryl, aralkyl, heterocyclylalkyl or heteroarylalkyl.

**12.** A method for the preparation of a compound of Formula IX,

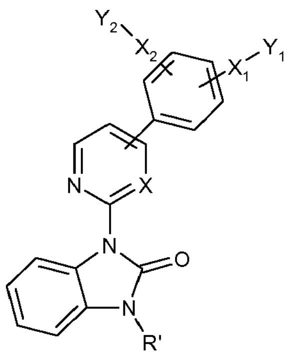

Formula IX

the method comprising

a. coupling of a compound of Formula II with a compound of Formula IIa to give a compound of Formula III,

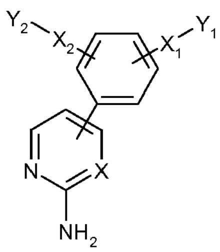
Formula II

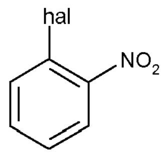
Formula IIa

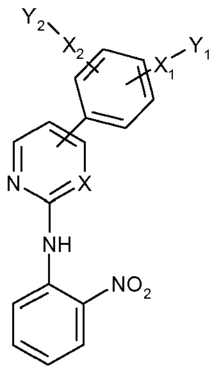
Formula III

b. carrying out reduction of the compound of Formula III to give a compound of Formula IV,

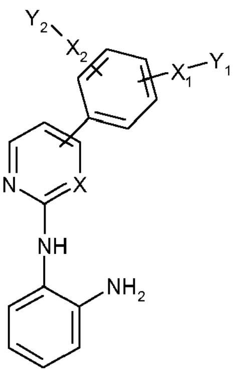
Formula IV

c. cyclizing the compound of Formula IV by reacting with a compound of Formula K to give a compound of Formula V,

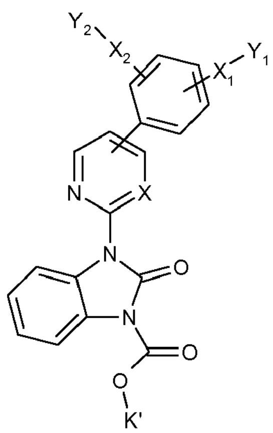
Formula V

d. deprotecting the compound of Formula V to give a compound of Formula VI, and

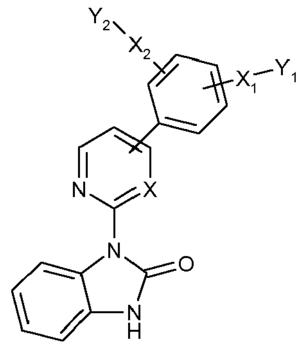

Formula VI

e. reacting the compound of Formula VI with a compound of Formula VIa

R'-hal Formula VIa

to give a compound of Formula IX,
wherein $Y_1$ and $Y_2$ independently are hydrogen, alkyl, cycloalkyl, heterocyclyl, heteroaryl, aralkyl, heterocyclylalkyl or heteroarylalkyl;
$X_1$ and $X_2$ independently are oxygen or sulphur;
X is nitrogen or carbon;
hal is fluorine, chlorine, bromine or iodine,
K is $(CO_2CH_2C_6H_5)_2O$, $(CO_2C(CH_3)_3)_2O$, $(CO_2C(CH_3)_2CHBr_2)_2O$ or $(CO_2C(CH_3)_2CCl_3)_2O$,
K' is $-CH_2C_6H_5$, $-C(CH_3)_3$, $-C(CH_3)_2CHBr_2$ or $-C(CH_3)_2CCl_3$ and
R' is alkyl, alkenyl, alkynyl, cycloalkyl, aryl, heteroaryl, heterocyclyl, aralkyl or heteroarylalkyl.

**13.** A method for the preparation of a compound of Formula XI,

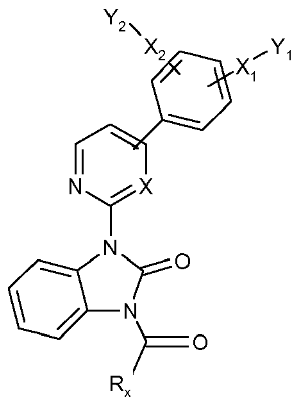

Formula XI

the method comprising

a. coupling of a compound of Formula II with a compound of Formula IIa to give a compound of Formula III,

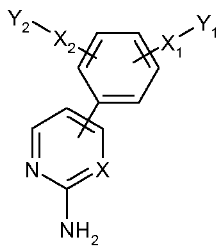

Formula II

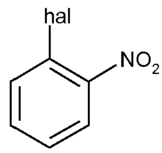

Formula IIa

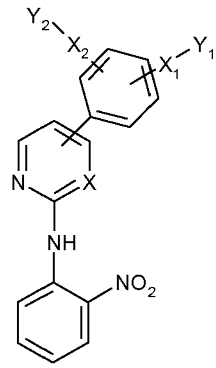

Formula III

b. carrying out reduction of the compound of Formula III to give a compound of Formula IV,

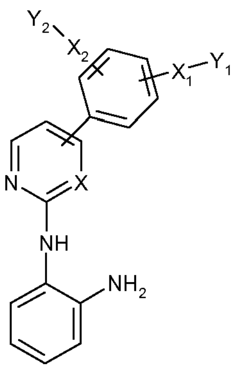

Formula IV

c. cyclizing the compound of Formula IV by reacting with a compound of Formula K to give a compound of Formula V,

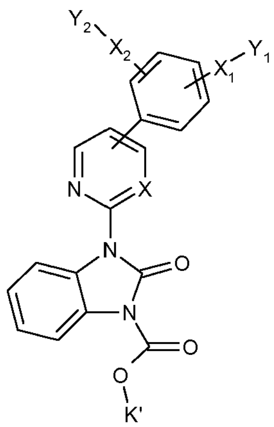

Formula V

d. deprotecting the compound of Formula V to give a compound of Formula VI,

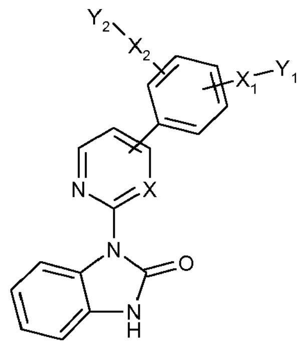

Formula VI

e. reacting the compound of Formula VI with a compound of Formula X

$(R_xCO)_2O$ Formula X

to give a compound of Formula XI,
wherein $Y_1$ and $Y_2$ independently are hydrogen, alkyl, cycloalkyl, heterocyclyl, heteroaryl, aralkyl, heterocyclylalkyl or heteroarylalkyl;
$X_1$ and $X_2$ independently are oxygen or sulphur;
X is nitrogen or carbon;
hal is fluorine, chlorine, bromine or iodine,
K is $(CO_2CH_2C_6H_5)_2O$, $(CO_2C(CH_3)_3)_2O$, $(CO_2C(CH_3)_2CHBr_2)_2O$ or $(CO_2C(CH_3)_2CCl_3)_2O$,
K' is $-CH_2C_6H_5$, $-C(CH_3)_3$, $-C(CH_3)_2CHBr_2$ or $-C(CH_3)_2CCl_3$ and
$R_x$ is hydrogen, alkyl, alkenyl, cycloalkyl, aryl, heterocyclyl, heteroaryl, aralkyl, heterocyclylalkyl or heteroarylalkyl.

**14.** A method for the preparation of a compound of Formula XII,

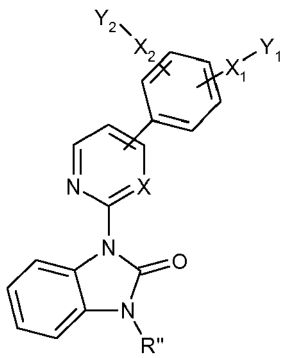

Formula XII

the method comprising

a. coupling of a compound of Formula II with a compound of Formula IIa to give a compound of Formula III,

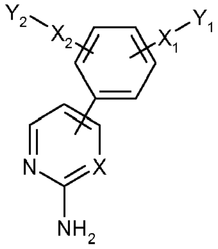

Formula II

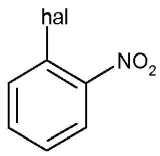

Formula IIa

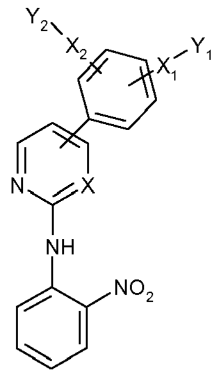

Formula III

b. carrying out reduction of the compound of Formula III to give a compound of Formula IV,

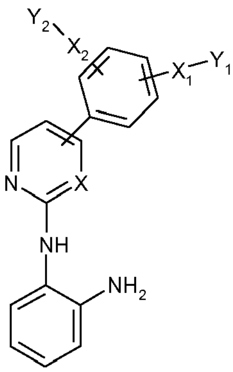

Formula IV

c. cyclizing the compound of Formula IV by reacting with a compound of Formula K to give a compound of Formula V,

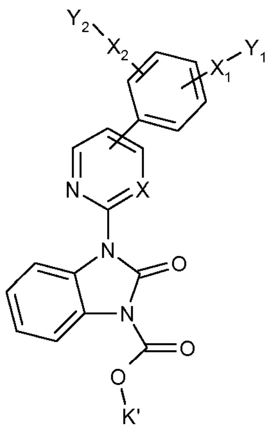

Formula V

d. deprotecting the compound of Formula V to give a compound of Formula VI,

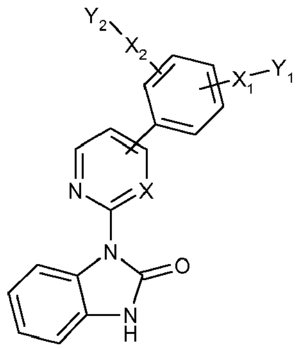

Formula VI

e. reacting the compound of Formula VI with a compound of Formula XIIa

R"COOR'''    Formula XIIa

to give a compound of Formula XII,
wherein $Y_1$ and $Y_2$ independently are hydrogen, alkyl, cycloalkyl, heterocyclyl, heteroaryl, aralkyl, heterocyclylalkyl or heteroarylalkyl;
$X_1$ and $X_2$ independently are oxygen or sulphur;
X is nitrogen or carbon;
hal is fluorine, chlorine, bromine or iodine,
K is $(CO_2CH_2C_6H_5)_2O$, $(CO_2C(CH_3)_3)_2O$, $(CO_2C(CH_3)_2CHBr_2)_2O$ or $(CO_2C(CH_3)_2CCl_3)_2O$,
K' is $-CH_2C_6H_5$, $-C(CH_3)_3$, $-C(CH_3)_2CHBr_2$ or $-C(CH_3)_2CCl_3$,
R" is haloalkyl and
R''' is alkyl.

European Patent Office

# PARTIAL EUROPEAN SEARCH REPORT

which under Rule 63 of the European Patent Convention shall be considered, for the purposes of subsequent proceedings, as the European search report

Application Number
EP 08 15 1539

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 01/05770 A (FUJISAWA PHARMACEUTICAL CO [JP]; SAWADA KOZO [JP]; INOUE TAKAYUKI [JP]) 25 January 2001 (2001-01-25)<br>* page 1, line 29 - page 2, line 5 *<br>* claim 1 *<br>----- | 1-14 | INV.<br>C07D401/04<br>C07D403/04<br>A61K31/4166<br>A61K31/4427<br>A61K31/506 |
| A | WO 02/069905 A (SQUIBB BRISTOL MYERS CO [US]; MACOR JOHN E [US]; CARLSON KENNETH E [US) 12 September 2002 (2002-09-12)<br>* page 57; example 28 *<br>* claim 7 *<br>----- | 1-14 | |
| A | WO 2006/031610 A (MERCK & CO INC [US]; BELL IAN M [US]; THEBERGE CORY R [US]; STUMP CRAI) 23 March 2006 (2006-03-23)<br>* examples 1-3 *<br>* claim 1 *<br>----- | 1-14 | |
| A | EP 0 748 805 A (TANABE SEIYAKU CO [JP]) 18 December 1996 (1996-12-18)<br>* page 24; example 55 *<br>* claim 1 *<br>-----<br>-/-- | 1-14 | TECHNICAL FIELDS SEARCHED (IPC)<br>C07D<br>A61K |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

3

| Place of search | Date of completion of the search | Examiner |
|---|---|---|
| Munich | 10 June 2008 | Samsam Bakhtiary, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

European Patent Office

# PARTIAL EUROPEAN SEARCH REPORT

Application Number
EP 08 15 1539

| Category | Citation of document with indication, where appropriate, of relevant passages | Relevant to claim | CLASSIFICATION OF THE APPLICATION (IPC) |
|---|---|---|---|
| A | BIANCHI MARIO ET AL: "Compounds with antiulcer and antisecretory activity. II. 3-Heteroaryl-benzimidazolin-2-ones" CHIMIE THERAPEUTIQUE, EDITIONS DIMEO, ARCUEIL, FR, vol. 18, no. 6, 1 January 1983 (1983-01-01), pages 495-500, XP008089530 ISSN: 0009-4374 * page 497; table IV * ----- | 1-14 | |
| A | BELL ET AL: "Identification of novel, orally bioavailable spirohydantoin CGRP receptor antagonists" BIOORGANIC & MEDICINAL CHEMISTRY LETTERS, OXFORD, GB, vol. 16, no. 24, 12 November 2006 (2006-11-12), pages 6165-6169, XP005761926 ISSN: 0960-894X * page 6167; compounds 12,16,17 * ----- | 1-14 | TECHNICAL FIELDS SEARCHED (IPC) |

3

EPO FORM 1503 03.82 (P04C10)

European Patent Office

# INCOMPLETE SEARCH SHEET C

Application Number
EP 08 15 1539

Although claims 5-8 are directed to a method of treatment of the human/animal body (Article 53(c) EPC), the search has been carried out and based on the alleged effects of the compound/composition.

-----

Claim(s) not searched:
-

Reason for the limitation of the search (non-patentable invention(s)):

Article 53 (c) EPC - Method for treatment of the human or animal body by therapy

## ANNEX TO THE EUROPEAN SEARCH REPORT ON EUROPEAN PATENT APPLICATION NO. EP 08 15 1539

This annex lists the patent family members relating to the patent documents cited in the above-mentioned European search report.
The members are as contained in the European Patent Office EDP file on
The European Patent Office is in no way liable for these particulars which are merely given for the purpose of information.

10-06-2008

| Patent document cited in search report | Publication date | Patent family member(s) | Publication date |
|---|---|---|---|
| WO 0105770 A | 25-01-2001 | BR 0013041 A | 16-07-2002 |
| | | CA 2379554 A1 | 25-01-2001 |
| | | CN 1374952 A | 16-10-2002 |
| | | CZ 20020233 A3 | 17-04-2002 |
| | | EP 1196391 A1 | 17-04-2002 |
| | | HU 0202186 A2 | 28-12-2002 |
| | | JP 2003505376 T | 12-02-2003 |
| | | MX PA02000340 A | 21-06-2002 |
| | | TR 200200161 T2 | 21-05-2002 |
| | | US 6582351 B1 | 24-06-2003 |
| WO 02069905 A | 12-09-2002 | CA 2439691 A1 | 12-09-2002 |
| | | EP 1370211 A2 | 17-12-2003 |
| | | JP 2005506286 T | 03-03-2005 |
| WO 2006031610 A | 23-03-2006 | AU 2005285109 A1 | 23-03-2006 |
| | | CA 2579847 A1 | 23-03-2006 |
| | | CN 101018781 A | 15-08-2007 |
| | | EP 1797073 A2 | 20-06-2007 |
| | | JP 2008512481 T | 24-04-2008 |
| | | US 2008096878 A1 | 24-04-2008 |
| EP 0748805 A | 18-12-1996 | AR 002483 A1 | 25-03-1998 |
| | | AT 164843 T | 15-04-1998 |
| | | AU 706156 B2 | 10-06-1999 |
| | | AU 5469396 A | 02-01-1997 |
| | | BG 62521 B1 | 31-01-2000 |
| | | BG 100656 A | 30-09-1997 |
| | | BR PI9602802 A | 06-10-1998 |
| | | CA 2178974 A1 | 16-12-1996 |
| | | CN 1142497 A | 12-02-1997 |
| | | DE 69600221 D1 | 14-05-1998 |
| | | DE 69600221 T2 | 06-08-1998 |
| | | ES 2116131 T3 | 01-07-1998 |
| | | HK 1010116 A1 | 11-06-1999 |
| | | HU 9601652 A1 | 29-09-1997 |
| | | IL 118469 A | 13-08-2000 |
| | | NO 962527 A | 16-12-1996 |
| | | RU 2129120 C1 | 20-04-1999 |
| | | SG 47152 A1 | 20-03-1998 |
| | | US 6005106 A | 21-12-1999 |
| | | ZA 9604652 A | 12-12-1996 |



For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0119798 A
- WO 2005016862 A
- WO 0276946 A
- WO 2004064835 A
- WO 0034248 A
- WO 0066564 A
- WO 9308180 A
- WO 2004096130 A
- WO 0214275 A
- US 3705233 A
- US 3644353 A
- US 3642896 A
- US 3700681 A
- US 4579985 A
- US 3994974 A
- US 3937838 A
- US 4419364 A
- US 5126375 A
- US 5243076 A
- US 4992474 A
- US 4011258 A
- US 3312590 A
- US 3983233 A
- US 3929768 A
- US 3721687 A
- US 3436389 A
- US 3506694 A
- US 3639434 A
- US 3992534 A
- US 3928326 A
- US 3980778 A
- US 3780177 A
- US 3652554 A
- US 3947478 A
- US 4076708 A
- US 4124707 A
- US 4158055 A
- US 4298604 A
- US 4335121 A
- US 4081541 A
- US 4226862 A
- US 4290962 A
- US 4587236 A
- US 4472392 A
- US 4472393 A
- US 4242334 A
- US 4014909 A
- US 4098803 A
- US 4619921 A
- US 5482934 A
- US 5837699 A
- US 5889015 A
- US 5278156 A
- US 5015746 A
- US 5976573 A
- US 6337324 A
- US 6057307 A
- US 6723713 A
- US 6127353 A
- US 6180781 A
- WO 0232899 A
- WO 0232898 A
- WO 06021848 A
- WO 2006016237 A
- WO 2006056863 A
- WO 2006117657 A
- WO 2006082492 A
- US 5565473 A
- US 5583152 A
- US 4859692 A
- US 4780469 A
- US 4826868 A
- US 4873259 A
- EP 0419049 A
- EP 0542356 A
- EP 0542355 A
- EP 0287436 A
- EP 0389359 A
- EP 0988292 A
- WO 0226723 A
- WO 0190106 A


### Non-patent literature cited in the description


- **SUTHERLAND et al.** *Pharmacol. Rev.,* 1960, vol. 12, 265
- **BEAVO et al.** *TIPS,* 1990, vol. 11, 150
- **NICHOLUS et al.** *TIPS,* 1991, vol. 12, 19
- **VERGHESE.** *J. Mol. Cell. Cardiol,* 1989, vol. 12 (II), 61